# DEMANDE DE BREVET EUROPEEN

(11) **EP 2 267 137 A2**
(43) Date de publication de la demande: **29.12.2010**
(21) Numéro de dépôt: 10180608.1
(22) Date de dépôt: 27.01.2006
(51) Int. Cl.: C12N 15/82, C12N 15/11, C12N 9/10

(54) **Sytèem de production de terpènoides dans les plantes .**

(30) Priorité: 27.01.2005 FR 0500855
(62) Demande divisionnaire de: 06709184.3
(71) Demandeur: Librophyt, F-84120 Pertuis (FR)
(72) Inventeur: TISSIER, Alain, 34070, MONTPELLIER (FR); SALLAUD, Christophe, 34070, MONTPELLIER (FR); RONTEIN, Denis, 04800, GREOUX LES BAINS (FR)
(74) Mandataire: Gallois, Valérie

(57) **Abrégé**

La présente invention se rapporte à une méthode de production de terpènes d'intérêt dans des plantes présentant des trichomes glanduleux, ainsi qu'aux plantes utiles pour la production de ces terpènes d'intérêt. Ces plantes comprennent une séquence codant pour une terpène synthase hétérologue sous contrôle d'un promoteur permettant son expression spécifique dans les trichomes. En outre, la voie de production de diterpène endogène est de préférence bloquée dans les trichomes des plantes, pour augmenter le flux dans la voie hétérologue. La sécrétion des terpènes hétérologues est spontanée résultant en une collecte facilitée. La présente invention concerne également des plantes présentant un blocage de la production d'un composé ayant des propriétés antibiotiques à la surface des feuilles montrant une efficacité augmentée de transformation par une bactérie.

SEQUENCE LISTING

<110>  LIBROPHYT

<120>  SYSTEME DE PRODUCTION DE TERPENOIDES DANS LES PLANTES

<130>  B0334WO

<150>  FR 05 00855
<151>  2005-01-27

<160>  9

<170>  PatentIn version 3.1

<210>  1
<211>  4720
<212>  DNA
<213>  artificial sequence

<220>
<223>  Construction de la Figure 1
<400>  1
gaattccaat cccacaaaaa tctgagctta acagcacagt tgctcctctc agagcagaat      60
cgggtattca acaccctcat atcaactact acgttgtgta taacggtcca catgccggta     120
tatacgatga ctggggttgt acaaaggcag caacaaacgg cgttcccgga gttgcacaca     180
agaaatttgc cactattaca gaggcaagag cagcagctga cgcgtataca acaagtcagc     240
aaacagatag gttgaacttc atccccaaag gagaagctca actcaagccc aagagctttg     300
ctaaggccct aacaagccca ccaaagcaaa aagcccactg gctcacgcta ggaaccaaaa     360
ggcccagcag tgatctagcc ccaaaagaga tctcctttgc cccggagatt acaatggacg     420
acttcctcta tctctacgat ctaggaagaa agttcgacgg tgaaggtgac gacaccatgt     480
tcaccactga taatgagaag attagcctct tcaatttcag aaagaatgct gacccacaga     540
tggttagaga ggcctacgca gcaggcctca tcaagacgat ctacccgagt aacaatctcc     600
aggagatcaa ataccttccc aagaaggtta aagatgcagt caaaagattc aggactaact     660
gcatcaagaa cacagagaaa gatatatttc tcaagatcag aagtactatt ccagtatgga     720

cgattcaagg cttgcttcat aaaccaaggc aagtaataga gattggagtc tctaaaaagg 780
tagttcctac tgaatcaaag gccatggagt caaagattca aatagaggac ctaacagaac 840
tcgccgtgaa aactggcgaa cagttcatac agagtctttt acgactcaat gacaagaaga 900
aaatcttcgt caacatggtg gagcacgaca ctcttgtcta ctccaaaaat atcaaagata 960
cagtctcaga agaccaaagg gctattgaga cttttcaaca aagggtaatt tcgggaaacc 1020
tccttggatt ccattgccca gctatctgtc acttcatcga aaggacagta gaaaaggaag 1080
gtggctccta caaatgccat cattgcgata aaggaaaggc tatcgttcaa gatgcctctg 1140
ccgacagtgg tcccaaagat ggacccccac ccacgaggag catcgtggaa aaagaagacg 1200
ttccaaccac gtcttcaaag caagtggatt gatgtgacat ctccactgac gtaagggatg 1260
acgcacaatc ccactatcct tcgcaagacc cttcctctat ataaggaagt tcatttcatt 1320
tggagaggac acgctcgagt ataagagctc tatttttaca acaattacca acaacaacaa 1380
acaacaaaca acattacaat tacatttaca attaccatgg ctcagctctc atttaatgca 1440
gcgctgaaga tgaacgcatt ggggaacaag gcaatccacg atccaacgaa ttgcagagcc 1500
aaatctgagc gccaaatgat gtgggtttgc tccagatcag ggcgaaccag agtaaaaatg 1560
tcgagaggaa gtggtggtcc tggtcctgtc gtaatgatga gcagcagcac tggcactagc 1620
aaggtggttt ccgagacttc cagtaccatt gtggatgata tccctcgact ctccgccaat 1680
tatcatggcg atctgtggca ccacaatgtt atacaaactc tggagacacc gtttcgtgag 1740
agttctactt accaagaacg ggcagatgag ctggttgtga aaattaaaga tatgttcaat 1800
gcgctcggag acggagatat cagtccgtct gcatacgaca ctgcgtgggt ggcgaggctg 1860
gcgaccattt cctctgatgg atctgagaag ccacggtttc ctcaggccct caactgggtt 1920
ttcaacaacc agctccagga tggatcgtgg ggtatcgaat cgcactttag tttatgcgat 1980
cgattgctta acacgaccaa ttctgttatc gccctctcgg tttggaaaac agggcacagc 2040
caagtacaac aaggtgctga gtttattgca gagaatctaa gattactcaa tgaggaagat 2100
gagttgtccc cggatttcca aataatcttt cctgctctgc tgcaaaaggc aaaagcgttg 2160
gggatcaatc ttccttacga tcttccattt atcaaatatt tgtcgacaac acgggaagcc 2220
aggcttacag atgtttctgc ggcagcagac aatattccag ccaacatgtt gaatgcgttg 2280
gaaggtctcg aggaagttat tgactggaac aagattatga ggtttcaaag taaagatgga 2340
tctttcctga gctcccctgc ctccactgcc tgtgtactga tgaatacagg ggacgaaaaa 2400
tgtttcactt ttctcaacaa tctgctcgac aaattcggcg gctgcgtgcc ctgtatgtat 2460
tccatcgatc tgctggaacg cctttcgctg gttgataaca ttgagcatct cggaatcggt 2520
cgccatttca aacaagaaat caaaggagct cttgattatg tctacagaca ttggagtgaa 2580
aggggcatcg gttggggcag agacagcctt gttccagatc tcaacaccac agccctcggc 2640
ctgcgaactc ttcgcatgca cggatacaat gtttcttcag acgttttgaa taatttcaaa 2700
gatgaaaacg ggcggttctt ctcctctgcg ggccaaaccc atgtcgaatt gagaagcgtg 2760
gtgaatcttt tcagagcttc cgaccttgca tttcctgacg aaagagctat ggacgatgct 2820

agaaaatttg cagaaccata tcttagagag gcacttgcaa cgaaaatctc aaccaataca 2880

aaactattca aagagattga gtacgtggtg gagtaccctt ggcacatgag tatcccacgc 2940

ttagaagcca gaagttatat tgattcatat gacgacaatt atgtatggca gaggaagact 3000

ctatatagaa tgccatcttt gagtaattca aaatgtttag aattggcaaa attggacttc 3060

aatatcgtac aatctttgca tcaagaggag ttgaagcttc taacaagatg gtggaaggaa 3120

tccggcatgg cagatataaa tttcactcga caccgagtgg cggaggttta tttttcatca 3180

gctacatttg aacccgaata ttctgccact agaattgcct tcacaaaaat tggttgttta 3240

caagtccttt ttgatgatat ggctgacatc tttgcaacac tagatgaatt gaaaagtttc 3300

actgagggag taaagagatg ggatacatct ttgctacatg agattccaga gtgtatgcaa 3360

acttgcttta aagtttggtt caaattaatg gaagaagtaa ataatgatgt ggttaaggta 3420

caaggacgtg acatgctcgc tcacataaga aaaccctggg agttgtactt caattgttat 3480

gtacaagaaa gggagtggct tgaagccggg tatataccaa cttttgaaga gtacttaaag 3540

acttatgcta tatcagtagg ccttggaccg tgtaccctac aaccaatact actaatgggt 3600

gagcttgtga aagatgatgt tgttgagaaa gtgcactatc cctcaaatat gtttgagctt 3660

gtatccttga gctggcgact aacaaacgac accaaaacat atcaggctga aaaggctcga 3720

ggacaacaag cctcaggcat agcatgctat atgaaggata atccaggagc aactgaggaa 3780

gatgccatta agcacatatg tcgtgttgtt gatcgggcct tgaaagaagc aagctttgaa 3840

tatttcaaac catccaatga tatcccaatg ggttgcaagt cctttatttt taaccttaga 3900

ttgtgtgtcc aaatctttta caagtttata gatgggtacg gaatcgccaa tgaggagatt 3960

aaggactata taagaaaagt ttatattgat ccaattcaag tatgaggatc ctctagagtc 4020

ctgctttaat gagatatgcg agacgcctat gatcgcatga tatttgcttt caattctgtt 4080

gtgcacgttg taaaaaacct gagcatgtgt agctcagatc cttaccgccg gtttcggttc 4140

attctaatga atatatcacc cgttactatc gtatttttat gaataatatt ctccgttcaa 4200

tttactgatt gtaccctact acttatatgt acaatattaa aatgaaaaca atatattgtg 4260

ctgaataggt ttatagcgac atctatgata gagcgccaca ataacaaaca attgcgtttt 4320

attattacaa atccaatttt aaaaaaagcg gcagaaccgg tcaaacctaa aagactgatt 4380

acataaatct tattcaaatt tcaaaagtgc cccaggggct agtatctacg acacaccgag 4440

cggcgaacta ataacgctca ctgaagggaa ctccggttcc ccgccggcgc gcatgggtga 4500

gattccttga agttgagtat tggccgtccg ctctaccgaa agttacgggc accattcaac 4560

ccggtccagc acggcggccg ggtaaccgac ttgctgcccc gagaattatg cagcattttt 4620

ttggtgtatg tgggccccaa atgaagtgca ggtcaaacct tgacagtgac gacaaatcgt 4680

tgggcgggtc cagggcgaat tttgcgacaa cagtctgcag 4720

<210> 2

<211> 4225

<212>  DNA
<213>  artificial sequence

<220>
<223>  Construction de la Figure 2
<220>
<221>  misc_feature
<222>  (4028)..(4028)
<223>  n = a, t, g or c

<400>  2
gaattcaatt tatttttgta aaacttctct aatttttgga caaactctta tattgatttt      60
ttaatcgaag ccaaaatatt tatttaacta tgaaaaaatt ttaacaacta atttattatg     120
gtaaataata ttgatatggt aacttcaagc acatgacaaa aattataact aactgcagaa     180
gtttattgtc tctctgaatc ttgtggctat atcataacaa atacttgtag ctaataagcc     240
aacgatgttc tcggtttcat ataatttgaa ttttaaaata gtttttaaat ttaatattta     300
tttcaaatca ttattgtggc taacatgtta taatcgcagt aatatttgga gatgcaatac     360
ttatatttag ctacaaaatt ttattgtatc agaataagtt tgtagctatt aagttagttt     420
ttgccacaaa tttttataat tgaagcaaaa atacttattc agctacagta ttttgtatcg     480
agtaatattt tgtgactaga agattaatat tgctacagta atttcagacg tgtggcaaaa     540
actcataatt agctacaaaa tattgtcgta gcaataattt tttatatcta ttaatgcaat     600
tattactaca tgcttttata acttgaggca aaaatatcta atagctataa cattttgtta     660
gaagtaattt ttgtggctat aaaattggta ttgctacagt aatttcaaat gcgtggcaaa     720
aaaatacgat taactacgaa attttattgt agcaataact ttgtagctat ttgggtaata     780
ttgctacgac agttagcaat tatagcaaaa atgccaaatc agctttgtca atttaatttt     840
gtagctaatt tttttatgaa attgtaaata gctatgaaat tttaattttt gtggctattg     900
tcaggtatta gccacatata gctaagaatt tgtagctata tatacataat gttgtagtgg     960
caaattctaa cattgtacgc ttggctgccc tttttttttt ttttggctac aaaactctaa    1020
agtaaaggaa ctagaaaact cgtttggcga gagaaagaga gagccatggc tcagctctca    1080
tttaatgcag cgctgaagat gaacgcattg gggaacaagg caatccacga tccaacgaat    1140
tgcagagcca aatctgagcg ccaaatgatg tgggtttgct ccagatcagg gcgaaccaga    1200
gtaaaaatgt cgagaggaag tggtggtcct ggtcctgtcg taatgatgag cagcagcact    1260
ggcactagca aggtggtttc cgagacttcc agtaccattg tggatgatat ccctcgactc    1320
tccgccaatt atcatggcga tctgtggcac cacaatgtta tacaaactct ggagacaccg    1380
tttcgtgaga gttctactta ccaagaacgg gcagatgagc tggttgtgaa aattaaagat    1440

atgttcaatg cgctcggaga cggagatatc agtccgtctg catacgacac tgcgtgggtg 1500

gcgaggctgg cgaccatttc ctctgatgga tctgagaagc cacggtttcc tcaggccctc 1560

aactgggttt tcaacaacca gctccaggat ggatcgtggg gtatcgaatc gcactttagt 1620

ttatgcgatc gattgcttaa cacgaccaat tctgttatcg ccctctcggt ttggaaaaca 1680

gggcacagcc aagtacaaca aggtgctgag tttattgcag agaatctaag attactcaat 1740

gaggaagatg agttgtcccc ggatttccaa ataatctttc ctgctctgct gcaaaaggca 1800

aaagcgttgg ggatcaatct tccttacgat cttccattta tcaaatattt gtcgacaaca 1860

cgggaagcca ggcttacaga tgtttctgcg gcagcagaca atattccagc caacatgttg 1920

aatgcgttgg aaggtctcga ggaagttatt gactggaaca agattatgag gtttcaaagt 1980

aaagatggat ctttcctgag ctcccctgcc tccactgcct gtgtactgat gaatacaggg 2040

gacgaaaaat gtttcacttt tctcaacaat ctgctcgaca aattcggcgg ctgcgtgccc 2100

tgtatgtatt ccatcgatct gctggaacgc ctttcgctgg ttgataacat tgagcatctc 2160

ggaatcggtc gccatttcaa acaagaaatc aaaggagctc ttgattatgt ctacagacat 2220

tggagtgaaa ggggcatcgg ttggggcaga gacagccttg ttccagatct caacaccaca 2280

gccctcggcc tgcgaactct tcgcatgcac ggatacaatg tttcttcaga cgttttgaat 2340

aatttcaaag atgaaaacgg gcggttcttc tcctctgcgg gccaaaccca tgtcgaattg 2400

agaagcgtgg tgaatctttt cagagcttcc gaccttgcat ttcctgacga aagagctatg 2460

gacgatgcta gaaaatttgc agaaccatat cttagagagg cacttgcaac gaaaatctca 2520

accaatacaa aactattcaa agagattgag tacgtggtgg agtacccttg gcacatgagt 2580

atcccacgct tagaagccag aagttatatt gattcatatg acgacaatta tgtatggcag 2640

aggaagactc tatatagaat gccatctttg agtaattcaa aatgtttaga attggcaaaa 2700

ttggacttca atatcgtaca atctttgcat caagaggagt tgaagcttct aacaagatgg 2760

tggaaggaat ccggcatggc agatataaat ttcactcgac accgagtggc ggaggtttat 2820

ttttcatcag ctacatttga acccgaatat tctgccacta gaattgcctt cacaaaaatt 2880

ggttgtttac aagtcctttt tgatgatatg gctgacatct ttgcaacact agatgaattg 2940

aaaagtttca ctgagggagt aaagagatgg gatacatctt tgctacatga gattccagag 3000

tgtatgcaaa cttgctttaa agtttggttc aaattaatgg aagaagtaaa taatgatgtg 3060

gttaaggtac aaggacgtga catgctcgct cacataagaa aaccctggga gttgtacttc 3120

aattgttatg tacaagaaag ggagtggctt gaagccgggt atataccaac ttttgaagag 3180

tacttaaaga cttatgctat atcagtaggc cttggaccgt gtaccctaca accaatacta 3240

ctaatgggtg agcttgtgaa agatgatgtt gttgagaaag tgcactatcc ctcaaatatg 3300

tttgagcttg tatccttgag ctggcgacta acaaacgaca ccaaaacata tcaggctgaa 3360

aaggctcgag gacaacaagc ctcaggcata gcatgctata tgaaggataa tccaggagca 3420

actgaggaag atgccattaa gcacatatgt cgtgttgttg atcgggcctt gaaagaagca 3480

agctttgaat atttcaaacc atccaatgat atcccaatgg gttgcaagtc ctttattttt 3540

aaccttagat tgtgtgtcca aatcttttac aagtttatag atgggtacgg aatcgccaat 3600

gaggagatta aggactatat aagaaaagtt tatattgatc caattcaagt atgaggtacc 3660

ttatatataa caatgcagac acaccttcaa agctgagtat ttggagcaaa tatggaagca 3720

ttttgtattg tccatgtaac ctataagtca cgtgtttggg caatggcaac atttactaat 3780

atttgcatta tggtaggttg tttacatcac acctatcggg ggcgaccctt cctaaacctg 3840

acatgaatgt gtgatgctty gtgcacctgg cggctcattt ttactatttc actgttacaa 3900

cttatttgga cggttgttac ctattgaatc atgtagtatt gttacttgaa tacaatgttt 3960

attttaatta ttacttaaat tttattctat catatcgtta aatccatcat tacgtaacaa 4020

tgaaaagngt cactttatgg aatgacggat ttagtgtggt ggtgtcattt ccttgcattt 4080

ttctctcatc ttgaccttcc ttattattag ataatcatct tttatcattt atcctacttt 4140

ttatacaata attctactcc atatcctatt ttttcttagt aatgttgcaa gtttattctt 4200

catattgtta gtgcgtattg gatcc 4225

<210> 3

<211> 3440

<212> DNA

<213> artificial sequence

<220>

<223> Construction de la Figure 3

<400> 3
gagctcaaag aggtgaaacc taatctagta tgcaaaccat gttaaattct caattgtttt 60

gatagataat gagttttctg ataattaata aattattaga taattaaagg accaaattta 120

tatgactttt gttttttatc atcttgatca tatatacaat gtaatggata caagcttata 180

gttgtataaa ttctatataa ttagttattc atacattaat tagatatatt caattgttct 240

ttataaatat aattcaaacc tgaaagcaat acttattttg taagaattgc aatattgtta 300

ttttgttatg gacttaaata ttaaccatgt tataatctta agtttatatt attagaaaaa 360

cttagttttt gaaagactaa tatgaacatt agtacttatt tcaaaaataa gcgcttagat 420

atatgaaatt actttaagta cttatttaaa ataattaagt accacacata catacatatc 480

tctacaaact gttaaagttt tctatatgag tacttatttt aaaataagag cataaatata 540

ataaattatg ttaaattctt atttaaaata ataaaggacc aaacatgcat aaaataaagt 600

atgagcttaa taagtcaaga agctaattga taagcattga tgccaaatgc acttactaac 660

tgttctatat tgtaggaaaa atctaacttt tatattaaaa atttattttc ataaaacttc 720

cctaattttt gaacaaaatc ttatattgat tttttaatca aagccaaaat atttatttaa 780

ctatgaaaat tttttaacaa ctaatttatt atggtaaata atattgatat ggtaactttc 840

agcacatgac aaaaattata actaactgca gaagtttact gtctctctga atcttgtggc 900
tatgtcattc tatcataaca aatacttgta gctaatacgc caacgatgtt ctcgatttca 960
tataatttga attttaaaat agcttttaaa tttaatattt atttcaaatc attattgtga 1020
ctaacatgtt ataaccgcag taatatttgg agatgcaata cttatattta gctacaaaat 1080
tttattgtat cataataagt ttgtagctat taagttagtt tttgccacaa atttttataa 1140
ttgaagcaaa aatacctatt caactacaat attttgtatc gagtaatatt ttgtgactag 1200
aagattaata ttattacagt aatttctgac gtgtggcaaa aactcataat tatctacaaa 1260
atattgtcgt agcaataatt ttttatatct attaatccaa ttattgctac atgcttttat 1320
aacttgaggc aaaaatatct atttagctat aacattttgt tagaagtaat ttttgtgact 1380
ataaagttgt tattgctaca gtaatttcaa atgcgtggca aaaaaaatac gattagctac 1440
gaaattttat tgtagcaata aatttgtagc tatttgggta atattgctac gacagttagc 1500
aattatagca aaaatgctaa atcagctttg tcgatttaat tttgtagcta atttttttat 1560
gaatttgtaa atagctatga aattttaatt tttgtggcta ttgttaggta ttagccacat 1620
atagctaaga atttgtagct atatatacat aatgttgtag tggcaaattc taacattgta 1680
agcttggctg cctttttttt tttttgggct acaaaactct aaagtaaagg aactagaaaa 1740
ctcgtttggc gagagaaaga gggatccctg caggaaatta ctacccaaga gaaaaatgaa 1800
catgaaatgc taaaagaaat agttcggaaa atgttggtag aaactccaga taatagtaca 1860
caaaaactag tcttgattga cacaattcaa agattgggat tagcatatca tttcaatgat 1920
gagattgaaa actccattca aaacatcttt aatttgtctc aaaatagtga agatgacgat 1980
gaacacaacc tttatgttgc tgctcttcgt tttcgacttg cgaggcaaca aggatattac 2040
atgtcttcag gtaccttaca tttctgccct ttcccgcaca gcttcatttt ttttcgttgt 2100
taaaagacag ttcggcgcat aaaatatctc atgtatacgc agggtcagga cgaaccgccc 2160
ccaaggggtg taaagtatgc aacttaccct aatactaaat atctcgtgta tacacagggt 2220
caggacaagt cgcacccaag gggtgtaatg tagacaactt atcctaatgc tattagtaac 2280
tgattttatg gctcgaacac ataaattata ggtcacacag taacaacttt accgttgctc 2340
aaagactcgc cttcctcttt ttttagttat cgcaccttat ttgtgcagag aatagcaagt 2400
ttcgagatct gcttctatat agaagacttc tgtattatac ttttttattt tgtccttctg 2460
cttaaaaata gtaaaaaact atagtgtgga aattgtaaat ttcttaacta gctgtgaaat 2520
caaatagtta ttataggaat attatttaag actccactta tggaaaacca ctgggttgtt 2580
gttgttattg tcaataataa cttggggtac gatttacttc tttttccatg gcttgtccac 2640
gactatattc ctattaacaa tgttgtgact atgctttctt tgagtcgagg gtctattgat 2700
aacaggctct cgatctttac aaggtaaaag taatgtctgc gtacacactc tactccgcag 2760
actccacttg taggatttca ctgaatattt tttgttgttg ttgttgttgt aataacttag 2820
ggtttaattt cttgatgcta atgaaattca tttctttcaa aatataaaca tggtgttcaa 2880

ccagatgtgt tcaagcaatt cactaaccat gagtacttac ctgaagacat gtaatatcct 2940
tgttgcctcg caagtcgaaa acgaagagca gcaacataaa ggttgtgttc atcgtcatct 3000
tcactatttt gagacaaatt aaagatgttt tgaatggagt tttcaatctc atcattgaaa 3060
tgatatgcta atcccaatct ttgaattgtg tcaatcaaga ctagtttttg tgtactatta 3120
tctggagttt ctaccaacat tttccgaact atttctttta gcatttcatg ttcatttttc 3180
tcttgggtag taatttcctc gaggtttctt aagattgaat cctgttgccg gtcttgcgat 3240
gattatcata taatttctgt tgaattacgt taagcatgta ataattaaca tgtaatgcat 3300
gacgttattt atgagatggg tttttatgat tagagtcccg caattataca tttaatacgc 3360
gatagaaaac aaaatatagc gcgcaaacta ggataaatta tcgcgcgcgg tgtcatctat 3420
gttactagat cgggtctaga 3440

<210> 4
<211> 3442
<212> DNA
<213> artificial sequence

<220>
<223> Construction de la Figure 4
<220>
<221> misc_feature
<222> (3245)..(3245)
<223> n = a, t, g or c

<400> 4
gagctcaatt tatttttgta aaacttctct aatttttgga caaactctta tattgatttt 60
ttaatcgaag ccaaaatatt tatttaacta tgaaaaaatt ttaacaacta atttattatg 120
gtaaataata ttgatatggt aacttcaagc acatgacaaa aattataact aactgcagaa 180
gtttattgtc tctctgaatc ttgtggctat atcataacaa atacttgtag ctaataagcc 240
aacgatgttc tcggtttcat ataatttgaa ttttaaaata gtttttaaat ttaatattta 300
tttcaaatca ttattgtggc taacatgtta taatcgcagt aatatttgga gatgcaatac 360
ttatatttag ctacaaaatt ttattgtatc agaataagtt tgtagctatt aagttagttt 420
ttgccacaaa tttttataat tgaagcaaaa atacttattc agctacagta ttttgtatcg 480
agtaatattt tgtgactaga agattaatat tgctacagta atttcagacg tgtggcaaaa 540
actcataatt agctacaaaa tattgtcgta gcaataattt tttatatcta ttaatgcaat 600
tattactaca tgcttttata acttgaggca aaaatatcta atagctataa cattttgtta 660
gaagtaattt ttgtggctat aaaattggta ttgctacagt aatttcaaat gcgtggcaaa 720

aaaatacgat taactacgaa attttattgt agcaataact ttgtagctat ttgggtaata 780
ttgctacgac agttagcaat tatagcaaaa atgccaaatc agctttgtca atttaatttt 840
gtagctaatt tttttatgaa attgtaaata gctatgaaat tttaattttt gtggctattg 900
tcaggtatta gccacatata gctaagaatt tgtagctata tatacataat gttgtagtgg 960
caaattctaa cattgtacgc ttggctgccc tttttttttt ttttggctac aaaactctaa 1020
agtaaaggaa ctagaaaact cgtttggcga gagaaagaga gagccatggc attgccatca 1080
gctgctatgc aatccaaccc tgaaaagctt aacttatttc acagattgtc aagcttaccc 1140
accactagct tggaatatgg caataatcgc ttccctttct tttcctcatc tgccaagtca 1200
cactttaaaa aaccaactca agcatgttta tcctcaacaa cccaccaaga agttcgtcca 1260
ttagcatact ttcctcctac tgtctggggc aatcgctttg cttccttgac cttcaatcca 1320
tcggaatttg aatcgtatga tgaacgggta attgtgctga agaaaaaagt taaggacata 1380
ttaatttcat ctacaagtga ttcagtggag accgttattt taatcgactt attatgtcgg 1440
cttggcgtat catatcactt tgaaaatgat attgaagagc tactaagtaa aatcttcaac 1500
tcccagcctg accttgtcga tgaaaaagaa tgtgatctct acactgcggc aattgtattc 1560
cgagttttca gacagcatgg ttttaaaatg tcttcggatg tgtttagcaa attcaaggac 1620
agtgatggta agttcaagga atccctacgg ggtgatgcta agggtatgct cagccttttt 1680
gaagcttccc atctaagtgt gcatggagaa gacattcttg aagaagcctt tgctttcacc 1740
aaggattact tacagtcctc tgcagttgag ttattcccta atctcaaaag gcatataacg 1800
aacgccctag agcagccttt ccacagtggc gtgccgaggc tagaggccag gaaattcatc 1860
gatctatacg aagctgatat tgaatgccgg aatgaaactc tgctcgagtt tgcaaagttg 1920
gattataata gagttcagtt attgcaccaa caagagctgt gccagttctc aaagtggtgg 1980
aaagacctga atcttgcttc ggatattcct tatgcaagag acagaatggc agagattttc 2040
ttttgggcag tcgcgatgta ctttgagcct gactatgcac acacccgaat gattattgcg 2100
aaggttgtat tgcttatatc actaatagat gatacaattg atgcgtatgc aacaatggag 2160
gaaactcata ttcttgctga agcagtcgca aggtgggaca tgagctgcct cgagaagctg 2220
ccagattaca tgaaagttat ttataaacta ttgctaaaca ccttctctga attcgagaaa 2280
gaattgacgg cggaaggcaa gtcctacagc gtcaaatacg gaagggaagc gtttcaagaa 2340
ctagtgagag gttactacct ggaggctgta tggcgcgacg agggtaaaat accatcgttc 2400
gatgactact tgtataatgg atccatgacc accggattgc ctctcgtctc aacagcttct 2460
ttcatgggag ttcaagaaat tacaggtctc aacgaattcc aatggctgga aactaatccc 2520
aaattaagtt atgcttccgg tgcattcatc cgacttgtca acgacttaac ttctcatgtg 2580
actgaacaac aaagaggaca cgttgcatct tgcatcgact gctatatgaa ccaacatgga 2640
gtttccaaag acgaagcagt caaaatactt caaaaaatgg ctacagattg ttggaaagaa 2700
attaatgaag aatgtatgag gcagagtcaa gtgtcagtgg gtcacctaat gagaatagtt 2760

aatctggcac gtcttacgga tgtgagttac aagtatggag acggttacac tgattcccag 2820
caattgaaac aatttgttaa gggattgttc gttgatccaa tttctatttg aggtacctta 2880
tatataacaa tgcagacaca ccttcaaagc tgagtatttg gagcaaatat ggaagcattt 2940
tgtattgtcc atgtaaccta taagtcacgt gtttgggcaa tggcaacatt tactaatatt 3000
tgcattatgg taggttgttt acatcacacc tatcgggggc gacccttcct aaacctgaca 3060
tgaatgtgtg atgcttygtg cacctggcgg ctcattttta ctatttcact gttacaactt 3120
atttggacgg ttgttaccta ttgaatcatg tagtattgtt acttgaatac aatgtttatt 3180
ttaattatta cttaaatttt attctatcat atcgttaaat ccatcattac gtaacaatga 3240
aaagngtcac tttatggaat gacggattta gtgtggtggt gtcatttcct tgcatttttc 3300
tctcatcttg accttcctta ttattagata atcatctttt atcatttatc ctacttttta 3360
tacaataatt ctactccata tcctattttt tcttagtaat gttgcaagtt tattcttcat 3420
attgttagtg cgtattgtcg ac 3442

<210> 5
<211> 1068
<212> DNA
<213> Nicotiana sylvestris

<400> 5
aatttatttt cataaaactt ctctaatttt tgaacaaaat cttatattga ttttttaatc 60
aaagccaaaa tatttattta actatgaaaa ttttttaaca actaatttat tatggtaaat 120
aatattgata tggtaacttc aagcacatga caaaaattat aactaactgc agaagtttac 180
tgtctctctg aatcttgtgg ctatgtcatt ctatcataac aaatacttgt agctaatacg 240
ccaacgatgt tctcgatttc atataatttg aattttaaaa tagcttttaa atttaatatt 300
tatttcaaat cattattgtg actaacatgt tataaccgca gtaatatttg gagatgcaat 360
acttatattt agctacaaaa ttttattgta tcataataag tttgtagcta ttaagttagt 420
ttttgccaca aatttttata attgaagcaa aaatacctat tcaactacaa tattttgtat 480
cgagtaatat tttgtgacta gaagattaat attattacag taatttcaga cgtgtggcaa 540
aaactcataa ttatctacaa aatattgtcg tagcaataat tttttatatc tattaatcca 600
attattgcta catgctttta taacttgagg caaaaatatc tatttagcta taacattttg 660
ttagaagtaa tttttgtgac tataaagttg ttattgctac agtaatttca aatgcgtggc 720
aaaaaaaata cgattagcta cgaaatttta ttgtagcaat aaatttgtag ctatttgggt 780
aatattgcta cgacagttag caattatagc aaaaatgcta aatcagcttt gtcgatttaa 840
ttttgtagct aattttttta tgaatttgta aatagctatg aaattttaat ttttgtggct 900
attgttaggt attagccaca tatagctaag aatttgtagc tatatataca taatgttgta 960
gtggcaaatt ctaacattgt aagcttggct gccttttttt ttttttggct acaaaactct 1020

aaagtaaagg aactagaaaa ctcgtttggc gagagaaaga gagagatg 1068

<210> 6

<211> 1060

<212> DNA

<213> Nicotiana sylvestris

<400> 6
aatttatttt tgtaaaactt ctctaatttt tggacaaact cttatattga ttttttaatc 60

aaagccaaaa tatttattta actatgaaaa aattttaaca actaatttat tatggtaaat 120

aatattgata tggtaacttc aagcacatga caaaaattat aactaactgc agaagtttat 180

tgtctctctg aatcttgtgg ctatatcata acaaatactt gtagctaata agccaacgat 240

gttctcggtt tcatataatt tgaattttaa aatagttttt aaatttaata tttatttcaa 300

atcattattg tggctaacat gttataatcg cagtaatatt tggagatgca atacttatat 360

ttagctacaa aattttattg tatcagaata agtttgtagc tattaagtta gtttttgcca 420

caaattttta taattgaagc aaaaatactt attcagctac agtattttgt atcgagtaat 480

attttgtgac tagaagatta atattgctac agtaatttca gacgtgtggc aaaaactcat 540

aattagctac aaaatattgt cgtagcaata attttttata tctattaatg caattattac 600

tacatgcttt tataacttga ggcaaaaata tctaatagct ataacatttt gttagaagta 660

atttttgtgg ctataaaatt ggtattgcta cagtaatttc aaatgcgtgg caaaaaaata 720

cgattaacta cgaaatttta ttgtagcaat aactttgtag ctatttgggt aatattgcta 780

cgacagttag caattatagc aaaaatgcta aatcagcttt gtcaatttaa ttttgtagct 840

aattttttta tgaaattgta aatagctatg aaattttaat ttttgtggct attgttaggt 900

attagccaca tatagctaag aatttgtagc tatatataca taatgttgta gtggcaaatt 960

ctaacattgt acgcttggct gccctttttt ttttttttgg ctacaaaact ctaaagtaaa 1020

ggaactagaa aactcgtttg gcgagagaaa gagagagatg 1060

<210> 7

<211> 1060

<212> DNA

<213> Nicotiana sylvestris

<400> 7
aatttatttt cgtaaaattt ctctaatttg gacaaactct tatattgatt tttttaatca 60

aagccaaaat atttatttaa ctatgaaaat tttttaacaa ctaatttatt atggtaaata 120

atattgatat ggtaacttca agcacatgat aaaaattata actaactgca gaagtttact 180

gtctctttga atcttgtggt tatatcattc tatcataaca aatacttgta gctaataagc 240
caacgatgtt ctcggtttca tataatttga attttaaaat agtttttaaa tttaatattt 300
atttcaaatt attattgtgg ctaacatgtt ataaccgcag taatatttgg agatgcaata 360
cttatattta gcttgaaaat tttattgtat cagaacaagt ttgtagctat taagttagtt 420
tttgccacaa atttttataa ttgaagcaaa aatacctatt cagctacagt attttgtatc 480
gagtaatatt ttgtgactag aagattaata ttgctacagt aatttcagac gtgtggcaaa 540
aactcataat tagctacaaa atattgtcgt agcaataatt ttttatatct attaatccaa 600
ttattgctac atgcttttat aacttgaggc aaaaatatct atttagctat aacattttat 660
taaaagtaat ttttgtggct ataaagttgt tattgctaca gtaatttcaa atgcgtggca 720
aaaaaaatac gattagctac gaaattttat tgtagcaata aatttgtagc tatttgggta 780
atattgctac gacagttagc aattatagca aaaatgctaa attagctttg ttaatttaat 840
tttgtagcta aactttttta tgaaatttta atttttgtgg ctattgatag gtattagcta 900
caattttcat atatgtagct aagaatttgt agctatatat acataatgtt gtagtggcaa 960
attctaacat tgtacgcttg gctgcccttt ttttttggct acaaaactct aaagtaaagg 1020
aactagaaaa ctcgtttggc gagagaaaga gagagagatg 1060

<210> 8
<211> 1087
<212> DNA
<213> Nicotiana sylvestris

<400> 8
aatttatttt cgtaaaattt ctctaatttt tggacaaact cttatattgg ttttttaatc 60
aaagccaaaa tatttattta actatgaaat tttgttgaac aactaattta ttatggtaaa 120
taatattgat atggtaactt caagcacatg acaaaaatta taactaactg cagaagttta 180
ctgtctctct gaatcttgtg gctatatcat tctatataac aaatacttgt agctaataag 240
ccaacgatgt tctcggtttc atataatttg aattttaaaa tagtttttaa atttaatatt 300
tatttcaaat cattattgtg gctagcatgt tataaccgca gtaatatttg gagatgcaat 360
acttatattt agctacaaaa ttttattgta tcagaataag tttgtaacta ttaagttagt 420
ttttgccaca aatttttata attgaagcaa aaatacctat tcagctacga tattttgtat 480
cgagtaatat tttgtgacta gaagattaat attgctagag taatttcaga cgtgtggcaa 540
aaactcataa ttagctacaa aatattgtcg tagcaataat tgtttatatc tattaatcca 600
attattgcta tatgctttta taacttgagg caaaaatatt tatttagcta taacattttg 660
ttagaagtaa tttttgtggc tataaagttg ttattgctac ggtaatttca aatgcgtggc 720
aaacaaatac gattagctac gaaattttat tgtagcaata aatttgtagc tatttgggta 780
atattgctac gacagttagc aattatagca aaaatgctaa attagctttg tcaatttaat 840

tttttagcta aattttttta taaaattgta aatagccatg aaattttaat ttttgtggct 900

attgttaggt attagccaca attttcatat atgtatctaa gaatttgtag ctatatatac 960

ataatgttgt agtggcaaat tctaacattg taagcttagc tgcccttttt tttttttttt 1020

tttggctaca aaactctaaa gtaaaggaac tagaaaactc gtttggcgag agaaagaggg 1080

atccatg 1087

<210> 9

<211> 630

<212> DNA

<213> artificial sequence

<220>

<223> enhancer

<400> 9
tacagtctca gaagaccaaa gggctattga gacttttcaa caaagggtaa tatcgggaaa 60

cctcctcgga ttccattgcc cagctatctg tcacttcatc aaaaggacag tagaaaagga 120

aggtggcacc tacaaatgcc atcattgcga taaaggaaag gctatcgttc aagatgcctc 180

tgccgacagt ggtcccaaag atggaccccc acccacgagg agcatcgtgg aaaaagaaga 240

cgttccaacc acgtcttcaa agcaagtgga ttgatgtgaa catggtggag cacgacactc 300

tcgtctactc caagaatatc aaagatacag tctcagaaga ccaaagggct attgagactt 360

ttcaacaaag ggtaatatcg ggaaacctcc tcggattcca ttgcccagct atctgtcact 420

tcatcaaaag gacagtagaa aaggaaggtg gcacctacaa atgccatcat tgcgataaag 480

gaaaggctat cgttcaagat gcctctgccg acagtggtcc caaagatgga cccccaccca 540

cgaggagcat cgtggaaaaa gaagacgttc caaccacgtc ttcaaagcaa gtggattgat 600

gtgatatctc cactgacgta agggatgacg 630

## Description

### DOMAINE DE L'INVENTION

L'invention se rapporte à une méthode de production de composés d'intérêt dans des plantes et aux plantes génétiquement modifiées préparées pour être utilisées dans cette méthode. L'invention se rapporte également à des plantes génétiquement modifiées permettant une meilleure efficacité de transformation.

### INTRODUCTION

Les trichomes sont des organes localisés à la surface des parties aériennes des plantes supérieures (cf revue Wagner *et al*., 2004). Ils prennent des formes variées et sont classés en deux grandes catégories. La première regroupe les poils, ou trichomes tecteurs, uni- ou pluri-cellulaires. Ils ne sécrètent pas, ou tout au moins pas en quantités appréciables, de substances vers l'extérieur. La seconde rassemble tous les trichomes, qualifiés de sécréteurs ou glanduleux, qui ont une capacité accrue de synthétiser et de sécréter vers l'extérieur des substances variées. Dans cette catégorie, il existe plusieurs types de trichomes sécréteurs. On distinguera notamment les trichomes peltés, de la famille des Lamiacées par exemple (menthe, basilic, lavande, thym, etc.) et les trichomes glanduleux présents entre autres dans les familles des Solanacées (tomate, tabac, pomme de terre, poivron, aubergine, etc.), des Asteracées (tournesol, etc.), et des Cannabacées (ex: *Cannabis sativa*).

Les trichomes peltés des Lamiacées sont le siège de production de molécules volatiles, tels les monoterpènes (ex: menthol, terpineol). Leur structure est caractérisée par une poche huileuse située entre la membrane périplasmique apicale des cellules sécrétrices et une paroi, dans laquelle s'accumulent les essences volatiles (Turner *et al*., 2000). C'est lors de la rupture de ces poches, par exemple lorsque la feuille est froissée, que les essences sont libérées.

Les trichomes qualifiés de sécréteurs synthétisent préférentiellement des molécules peu ou pas volatiles à température ambiante, comme les sesquiterpènes ou les diterpènes (Wagner *et al*., 2004). Le tabac cultivé *(Nicotiana tabacum*) par exemple, produit au niveau de ses trichomes glanduleux, une sécrétion dont plus de la moitié est composée de diterpènes appartenant à deux classes, les cembranes et les labdanes (Heemann *et al.,* 1983). Chez certaines espèces de tabac sauvage comme *Nicotiana sylves tris,* seuls les cembranoïdes, et plus particulièrement le cembratriène-diol (CBT-diol), sont présents et les quantités accumulées à la surface des feuilles sont de l'ordre de 15% de la masse sèche de la feuille (Severson *et al.,* 1985).

Tous les diterpènes ont pour origine le même substrat de départ, le géranylgéranyldiphosphate (GGPP). Ce qui fait la diversité des diterpènes, ce sont les diterpènes synthases qui utilisent le GGPP pour en faire une oléfine, cyclique ou non. Le GGPP est également le précurseur des tétraterpènes, parmi lesquels on trouve les pigments caroténoïdes. Parmi les diterpènes, on compte des molécules du métabolisme primaire, comme les gibbérellines, hormones nécessaires à la croissance des plantes, et des métabolites secondaires qui représentent la majorité de la diversité métabolique de ces molécules. Ce partage entre métabolites primaires et secondaires se traduit par une forte régulation de la disponibilité métabolique du GGPP. A ce titre, il est pertinent de constater que les espèces végétales qui accumulent des quantités importantes de diterpènes sont dotées d'organes spécialisés dédiés à leur synthèse, les trichomes sécréteurs. Les tabacs et en particulier *Nicotiana sylves tris,* sont un exemple caractéristique où le GGPP est hautement disponible dans les trichomes pour assurer un flux important de synthèse de cembranoïdes, et donc leur accumulation abondante à la surface des parties aériennes.

Les étapes conduisant à la biosynthèse du CBT-diol chez le tabac sont partiellement connues et peuvent se décomposer en deux parties distinctes:
- la biosynthèse du précurseur universel de tous les diterpènes, le géranylgéranyl pyrophosphate (GGPP), produit par la voie dite de « Rohmer » (Rohmer *et al.,* 1996), s'effectue dans le chloroplaste.
- La biosynthèse du CBT-diol s'effectue à partir du GGPP. Il a été proposé par Wang et Wagner (2003) le schéma de biosynthèse suivant :
   GGPP → CBT-ol → CBT-diol

La première étape de cyclisation serait réalisée par une enzyme appartenant à une large famille d'enzymes connues sous le nom de terpène synthases (Bohlmann *et al.,* 1998). La diterpène synthase de tabac utiliserait le GGPP comme substrat pour former du CBT-ol. La seconde étape permettant de produire du CBT-diol à partir du CBT-ol est une étape d'hydroxylation catalysée par une enzyme appartenant à la famille des cytochromes P450. L'équipe du Professeur G. Wagner (University of Kentucky) a identifié, par PCR soustractive, deux gènes candidats de *N. tabacum* pour chacune de ces étapes :
- une séquence présentant une forte similarité avec des séquences codant pour des terpènes synthases (CYC-2 ; N° Genbank AF401234. NID : AY495694).
- une séquence codant pour une enzyme de type cytochrome P450 (CYP71D16, NID :
   AF166332) (Wang *et al.,* 2001, Wang & Wagner 2003).

Des expériences d'extinction de l'expression de ces gènes par co-suppression et RNAi chez *N. tabacum* ont montré (i) une diminution de CBT-diol et de CBT-ol corrélée avec une diminution de l'expression du gène CYC-2, et (ii) une augmentation de l'accumulation de CBT-ol et une diminution de CBT-diol en corrélation avec une diminution de l'expression du gène CYP71D16 dans les trichomes. Ces travaux ont suggéré que (i) le gène CYC-2 code pour la CBT-ol cyclase responsable de la synthèse de CBT-ol et (ii) le gène CYP71D16 code pour une CBT-ol hydroxylase responsable de la synthèse de CBT-diol à partir du CBT-ol. Par ailleurs, une séquence génomique d'un gène très proche de l'ARNm CYC-2 a été récemment déposée dans les bases de données (CYC-1, NID: AY049090), ce qui suggère l'existence non pas d'un seul mais de plusieurs gènes de CBT-ol cyclases.

Certains diterpènoïdes sont l'objet d'une exploitation commerciale, notamment dans le secteur pharmaceutique. C'est le cas notamment des diterpènoïdes de la classe des taxanes, le paclitaxel et le docetaxel, utilisés dans le traitement des cancers du sein et de l'ovaire. Le paclitaxel est une molécule naturelle extraite de l'if *(Taxus sp.),* tandis que le docetaxel est une molécule semi-synthétique, dérivée d'un précurseur du paclitaxel, la 10-déacétyl baccatine III (ou 10-DAB III), également extraite de l'if. La plupart des gènes de biosynthèse du paclitaxel de l'if ont été décrits (Jennewein & Croteau, 2001 ; Jennewein *et al.,* 2004). La production de ces molécules reste coûteuse en raison de la relativement faible abondance de la 10-DAB III et surtout du paclitaxel dans les extraits d'if, et de l'absence de procédé de synthèse industrialisable, en raison de la complexité structurale des molécules.

Il existe donc une forte demande de procédés qui permettraient de produire à moindre coût des terpènes d'intérêt, mais aussi de produire des molécules dérivées de terpènes encore difficilement accessibles à la synthèse.

### RESUME DE L'INVENTION

La présente invention décrit une nouvelle méthode de production de terpènes d'intérêt dans des plantes présentant des trichomes glanduleux, ainsi que des plantes utiles pour cette production. Cette nouvelle méthode est basée sur l'introduction dans la plante d'une terpène synthase hétérologue permettant de produire le terpène d'intérêt. L'expression de cette terpène synthase hétérologue est contrôlée par un promoteur permettant une expression, de préférence spécifique, dans les trichomes glanduleux de la plante. Pour augmenter le rendement, il est préférable de rompre la voie de synthèse des diterpènes endogènes dans les trichomes de la plante.

Un premier objet de la présente invention concerne une méthode de production de terpène d'intérêt dans une plante présentant des trichomes glanduleux comprenant :
a) l'introduction dans une cellule de ladite plante d'une construction portant une cassette d'expression comprenant une séquence polynucléotidique codant pour une terpène synthase hétérologue permettant de synthétiser ledit terpène d'intérêt sous le contrôle d'un promoteur permettant une expression, de préférence spécifique, dans les trichomes;
b) la reconstitution d'une plante à partir de ladite cellule et la sélection des plantes transgéniques exprimant ladite terpène synthase ; et
c) la récolte du terpène d'intérêt contenu dans les trichomes des dites plantes transgéniques.

De préférence, l'expression de la terpène synthase est sous le contrôle d'un promoteur spécifique des trichomes.

De préférence, la récolte du terpène d'intérêt contenu dans les trichomes des dites plantes transgéniques est effectuée par la récupération du terpène d'intérêt contenu dans l'exsudat des trichomes.

Dans un mode de réalisation particulier préféré, ladite cassette d'expression comprend au moins une séquence activatrice « enhancer » liée de manière opérationnelle au promoteur. Dans un mode de réalisation particulier, la séquence activatrice comprend la séquence SEQ ID No 9.

De préférence, la méthode comprend en outre le blocage de la voie de production de diterpène endogène dans les trichomes. Plus particulièrement, le blocage de la voie de production de diterpène endogène peut être effectué en bloquant l'expression de la ou des diterpènes synthases endogènes. De préférence, le blocage de la voie de production des diterpènes endogènes dans les trichomes est réalisé en croisant la plante transgénique sélectionnée en b) avec une plante transgénique dont la voie de production de diterpènes endogènes est bloquée dans les trichomes. Dans un mode de réalisation particulier, une des diterpènes synthases endogènes est la cembratriène-ol synthase.

Dans un mode de réalisation préféré, la terpène synthase hétérologue est une diterpène synthase. De préférence, la diterpène synthase est la taxadiène synthase ou la casbène synthase.

Dans un autre mode de réalisation préféré, la terpène synthase hétérologue est une monoterpène synthase et la construction comprend en outre une séquence polynucléotidique codant pour une géranylpyrophosphate synthase sous le contrôle d'un promoteur permettant son expression dans les trichomes. Alternativement, la séquence polynucléotidique codant pour une géranylpyrophosphate synthase peut être portée par une deuxième construction distincte de la première.

Dans un mode de réalisation préféré supplémentaire, la terpène synthase hétérologue est une sesquiterpène synthase et la construction comprend en outre une séquence polynucléotidique codant pour une farnésylpyrophosphate synthase sous le contrôle d'un promoteur permettant son expression dans les trichomes. Alternativement, la séquence polynucléotidique codant pour une farnésylpyrophosphate synthase peut être portée par une deuxième construction distincte de la première.

Dans un mode de réalisation préféré additionnel, la terpène synthase hétérologue est une triterpène synthase et la construction comprend en outre des séquences polynucléotidiques codant pour une farnésylpyrophosphate synthase, une squalène synthase et une squalène époxydase sous le contrôle d'un promoteur permettant son expression dans les trichomes. Alternativement, les séquences polynucléotidiques codant pour la farnésylpyrophosphate synthase, la squalène synthase et la squalène époxydase peuvent être portées par une ou plusieurs constructions distinctes de la première.

Dans un mode de réalisation préféré, la plante présentant des trichomes glanduleux est une plante de la famille des Asteracées, Cannabacées, Solanacées ou Lamiacées. De préférence, la plante est le tabac, et plus particulièrement *Nicotiana sylvestris.*

Dans un deuxième aspect, la présente invention concerne une plante ou graine d'une plante transgènique présentant des trichomes glanduleux, **caractérisée en ce que** la voie de production de diterpène endogène est bloquée dans les trichomes. Plus particulièrement, le blocage de la voie de production de diterpène endogène peut être effectué en bloquant l'expression des diterpènes synthases endogènes. De préférence, la plante présentant des trichomes glanduleux est une plante de la famille des Asteracées, Cannabacées, Solanacées ou Lamiacées. De manière encore plus préférée, la plante est le tabac, et plus particulièrement *Nicotiana sylvestris.* Dans un mode de réalisation particulier, la diterpène synthase endogène est la cembratriène-ol synthase.

Dans un troisième aspect, la présente invention concerne une plante ou graine transgènique présentant des trichomes glanduleux **caractérisée en ce qu**'elle comprend une cassette d'expression contenant une séquence polynucléotidique codant pour une terpène synthase hétérologue permettant de synthétiser un terpène d'intérêt sous le contrôle d'un promoteur permettant une expression, de préférence spécifique, dans les trichomes. De préférence, l'expression de la terpène synthase est sous le contrôle d'un promoteur spécifique des trichomes. Dans un mode de réalisation préféré, ladite cassette d'expression comprend au moins une séquence activatrice « enhancer » liée de manière opérationnelle au promoteur. De préférence, la voie de production de diterpènes endogènes est bloquée dans les trichomes de ladite plante. Plus particulièrement, le blocage de la voie de production de diterpène endogène peut être effectué en bloquant l'expression de la diterpène synthase endogène. De préférence, la plante présentant des trichomes glanduleux est une plante de la famille des Asteracées, Cannabacées, Solanacées ou Lamiacées. De manière encore plus préférée, la plante est le tabac, et plus particulièrement *Nicotiana sylvestris.* Dans un mode de réalisation particulier, la diterpène synthase endogène est la cembratriène-ol synthase.

Un quatrième aspect de l'invention concerne l'utilisation d'une plante selon la présente invention pour la production de terpène d'intérêt.

Un cinquième aspect de la présente invention concerne une méthode de récolte de terpènes hétérologues dans l'exsudat des trichomes d'une plante, comprenant a) la récolte de parties aériennes de la plante ; b) l'incubation de ces parties aériennes dans un solvant de type peu polaire ou apolaire ; et c) l'élimination du solvant.

Un sixième aspect de la présente invention concerne en outre des plantes présentant une augmentation de l'efficacité de transformation par des bactéries permettant le transfert d'ADN dans des cellules végétales ainsi que leurs utilisations. Ainsi, la présente invention concerne une cellule d'une plante dont une voie de production d'un composé ayant une activité antibiotique à la surface des feuilles est bloquée pour transformer cette cellule avec une bactérie permettant le transfert d'ADN dans des cellules végétales. Elle concerne également une méthode de transformation d'une cellule végétale comprenant la mise en contact d'une bactérie permettant le transfert d'ADN dans des cellules végétales avec une cellule d'une plante dont une voie de production d'un composé ayant une activité antibiotique à la surface des feuilles est bloquée. Elle concerne en outre une méthode d'obtention de plantes transformées **caractérisé en ce qu**'il comprend les étapes suivantes : a) obtention d'une cellule hôte recombinante de bactérie permettant le transfert d'ADN dans des cellules végétales comprenant un transgène ; b) transformation d'une plante présentant un blocage de la production d'un composé ayant des propriétés antibiotiques à la surface des feuilles par infection avec les cellules hôtes recombinantes de bactéries obtenues à l'étape a) ; c) sélection des plantes ayant intégré dans leur génome le transgène. De préférence, la bactérie appartient aux genres Agrobacterium, notamment *Agrobacterium tumefaciens,* Rhizobium, Sinorhizobium, ou Mesorhizobium. De préférence, les plantes sont des plantes ayant des trichomes glanduleux. Dans un mode de réalisation préféré, la plante présentant des trichomes glanduleux est une plante de la famille des Asteracées, Cannabacées, Solanacées ou Lamiacées. Elle est de préférence de la famille des Solanacées et peut être sélectionnée parmi le tabac, la tomate, le tournesol et la pomme de terre. De préférence, le composé ayant une activité antibiotique est un terpène et notamment un diterpène. En particulier, le composé est le CBT-diol. De préférence, le blocage de la production de terpènes, en particulier de diterpènes tels que le CBT-diol, est réalisé en bloquant l'expression des terpènes synthases endogènes dans les trichomes, en particulier la cembratriène-ol synthase. Le blocage peut également être réalisé en bloquant spécifiquement dans les trichomes l'expression de la géranylgéranylpyrophosphate synthase (GGPPS), qui produit le géranylgéranylpyrophopshate, précurseur de tous les diterpènes.

Un septième aspect de la présente invention concerne l'utilisation d'une plante, dans laquelle la voie de production de diterpène endogène, en particulier de CBT-diol, est bloquée dans les trichomes, pour identifier la fonction de gènes de biosynthèse de terpènoïdes. Plus particulièrement, le blocage de la voie de production de CBT-diol peut être effectué en bloquant l'expression de la cembratriène-ol synthase.

### DESCRIPTION DETAILLEE DE L'INVENTION

Les inventeurs ont démontré que le tabac *Nicotiana sylves tris* par ses caractéristiques métaboliques précédemment évoquées, constitue un hôte idéal pour y greffer la voie de biosynthèse du taxol sur le pool de GGPP endogène. De manière plus générale, n'importe quelle terpène synthase, et plus particulièrement diterpène synthase, dès lors que sa séquence codante est connue, pourrait être intégrée dans le génome de tabac pour une production abondante des diterpènoïdes qui en sont dérivés. Ceci est généralisable pour d'autres classes de terpènes (monoterpènes, sesquiterpenes, ou triterpènes par exemple) aux plantes présentant des trichomes glanduleux.

L'invention est essentiellement constituée par trois éléments résumés ci-après.
(1) La manipulation génétique du tabac pour permettre l'expression *de novo* de la terpène synthase hétérologue dans les trichomes sécréteurs. Les exemples décrits ici sont ceux de la taxadiène synthase et de la casbène synthase afin de produire respectivement la taxadiène et la casbène. Ces exemples ne doivent pas être considérés comme limitatifs des possibilités de production de diterpènoïdes par les trichomes de tabac. Par ailleurs, l'importance de l'expression de diterpènes synthases de manière spécifique dans les trichomes de tabac par rapport à une expression constitutive sera soulignée.
(2) L'augmentation de la production de diterpènes par inhibition de la synthèse de diterpènes endogènes du tabac. Afin d'augmenter le niveau de production des diterpènes par les trichomes sécréteurs, la voix endogène de production de diterpènes est bloquée à l'étape de la terpène synthase. Ceci permet de diminuer, voir d'éliminer, la compétition pour le substrat commun à toutes les diterpènes synthases, le GGPP. L'inactivation des gènes codant l'activité de terpène synthase (cembratrièn-ol synthase dans le cas de *Nicotiana sylvestris)* est suffisante pour quasiment éliminer la production de diterpènes endogènes.
(3) La sécrétion dans l'exsudat des trichomes des diterpènes d'intérêt et la récolte de l'exsudat produit par les trichomes contenant des diterpènoïdes par un solvant de type peu polaire (chlorure de méthylène, chloroforme, etc.) ou apolaire (ex: pentane, hexane, etc.). En effet, les inventeurs ont constaté la possibilité non-évidente et imprévisible d'une sécrétion de terpènes autre que les terpènes endogènes dans l'exsudat des trichomes.

Les inventeurs ont également découvert que des plantes, dont la voie de production à la surface des feuilles d'un composé ayant une activité antibiotique est bloquée, présentent une efficacité plus élevée de transformation par des bactéries permettant le transfert d'ADN dans des cellules végétales. En effet, l'élimination de la production de CBT-diol produit par les trichomes à la surface des feuilles de *N. sylves tris* permet d'augmenter très significativement l'efficacité de transformation génétique par *Agrobacterium tumefaciens.* Ceci peut s'expliquer par l'activité antibactérienne du CBT-diol, qui empêcherait donc la croissance *d'Agrobacterium tumefaciens* et la transformation des cellules de *N. sylvestris.*

Par ailleurs, les inventeurs ont identifié plusieurs propriétés intéressantes des plantes dans lesquelles la voie de synthèse du CBT-diol est bloquée dans les trichomes. En effet, le CBT-diol est un contaminant majeur étant données les quantités qui sont produites. Sa présence rend difficile la détection de composés minoritaires. Ainsi, une plante ne produisant pas ou très peu de CBT-diol permet une purification plus facile des molécules produites dans les trichomes par transgenèse. En outre, une telle plante est extrêmement utile pour l'identification de la fonction de gènes de biosynthèse des terpènoïdes. En effet, le faible taux en CBT-diol dans les exsudats des feuilles d'une telle plante facilite l'identification « *in vivo »* de la fonction de gènes impliqués dans la biosynthèse des terpènes comme par exemple des terpènes synthases, des monooxygénases à cytochrome P450, des acétyltransférases, benzoyltransférases ou N-benzoyltransférases.

La présente invention consiste donc en un système de production de terpènes d'intérêt sélectionnés parmi les diterpènes, les monoterpènes, les sesquiterpènes et les triterpènes dans une plante possédant des trichomes glanduleux.

Dans un premier mode de réalisation, la présente invention consiste en un système de production de diterpènes d'intérêt, et plus particulièrement de taxanes, par les trichomes glanduleux.

Les expériences d'expression de diterpènes synthases dans *Nicotiana sylvestris* démontrent que les trichomes de tabac constituent une plateforme naturelle adaptée pour la production *de novo* de diterpènoïdes, et en particulier de taxadiène ou de casbène. La production de taxadiène ou de casbène dans l'exsudat des trichomes démontre que le système de sécrétion des trichomes n'est pas spécifique d'une classe de diterpènes. En vue d'une culture pour la production de ces diterpènes, l'expression spécifique de diterpène synthase dans les trichomes confère un avantage important sur l'expression constitutive qui est caractérisée par un retard de croissance.

Le choix du tabac et en particulier *Nicotiana sylvestris,* ainsi que les plantes présentant des trichomes glanduleux, est aussi bien adapté pour une production importante de diterpènes. En effet, l'expression de la taxadiène synthase sous un promoteur constitutif (35S) dans l'espèce *Arabidopsis thaliana* conduit à une accumulation de taxadiène limitée aux feuilles et dans des quantités faibles (100 fois inférieure à celle dans *N. sylvestris)* (Besumbes et al., 2004 ; Botella-Pavia et al., 2004). Cette différence est liée à la physiologie des trichomes d'*Arabidopsis thaliana* qui ne sont pas glanduleux.

Comme les inventeurs l'ont démontré, la casbène et la taxadiène synthase, qui sont issues de plantes phylogénétiquement éloignées (ricin et if respectivement), sont toutes les deux fonctionnelles dans les trichomes de tabacs. Suite à ces observations, les inventeurs estiment que les trichomes de tabac constitue une usine biologique capable d'exprimer toutes sortes de diterpènes synthases, quelles que soient leurs origines.

L'utilisation d'une stratégie d'extinction des gènes endogènes de terpènes synthases conduit à l'augmentation significative de l'accumulation de taxadiène par les trichomes. Cette stratégie est donc particulièrement adaptée à l'augmentation des taux de productions de diterpènes par les trichomes de tabac. De manière générale, l'extinction par quelque moyen que ce soit de la voie des diterpènoïdes endogènes du tabac pour augmenter la biosynthèse de la voie greffée par génie génétique, constitue un avantage évident pour une mise en production à haut rendement de diterpènoïdes d'intérêts.

De manière générale, une cassette d'expression est constituée d'un promoteur permettant d'initier la transcription, d'un acide nucléique transcrit, contenant ou non des introns, et dont la traduction permet la production d'une terpène synthase hétérologue, et d'un terminateur de transcription. Les acides nucléiques transcrits peuvent être des ADN génomiques, des ADN complémentaires (ADNc) ou des ADN synthétiques. Dans le cadre de la présente invention, les acides nucléiques transcrits sont de préférence des ADNc dénués d'introns. Les acides nucléiques transcrits peuvent être des molécules synthétiques ou semi-synthétiques, recombinantes, éventuellement amplifiées ou clonées dans des vecteurs, modifiées chimiquement ou comprenant des bases non-naturelles. Il s'agit typiquement de molécules d'ADN isolées, synthétisées par des techniques recombinantes bien connues en soi de l'homme du métier. Ils sont utilisés de préférence en pleine longueur, à savoir avec le codon initiateur ATG des gènes d'origine et avec leur séquence codante pour le peptide d'adressage vers les plastes. D'une manière générale, les diterpènes synthases de plantes sont adressées vers les plastes et donc possèdent un peptide d'adressage, alors que les diterpènes synthases d'organismes qui ne possèdent pas de plastes, comme les bactéries ou les champignons, n'ont pas de peptide d'adressage vers les plastes. Pour exprimer correctement ces diterpènes synthases dans les plastes de plantes et en particulier de tabac, il sera nécessaire de réaliser une fusion avec un peptide d'adressage, tel que celui de la petite sous-unité de la Rubisco, bien connu de l'homme du métier.

Le terme cassette d'expression désigne une construction d'acide nucléique comprenant une région codante et une région régulatrice, liées de manière opérationnelle. L'expression "lié de manière opérationnelle" indique que les éléments sont combinés de manière à ce que l'expression de la séquence codante (le gène d'intérêt) et/ou le ciblage de la protéine codée soient sous contrôle du promoteur transcriptionnel et/ou du peptide de transit. Typiquement, la séquence du promoteur est placée en amont du gène d'intérêt, à une distance de celui-ci compatible avec le contrôle de l'expression. De même, la séquence du peptide de transit est généralement fusionnée en amont de la séquence du gène d'intérêt, et en phase avec celui-ci, et en aval de tout promoteur. Des séquences d'espacement peuvent être présentes, entre les éléments régulateurs et le gène, dès lors qu'elles n'empêchent pas l'expression et/ou le ciblage.

La cassette d'expression comprend un promoteur permettant une expression, de préférence spécifique, dans les trichomes de la plante. De tels promoteurs sont connus par l'homme du métier.

Au sens de l'invention, on entend par promoteur "spécifique" un promoteur principalement actif dans un tissu ou un groupe cellulaire donné. Il est entendu qu'une expression résiduelle, généralement plus faible, dans d'autres tissus ou cellules ne peut être entièrement exclue. Une caractéristique particulière de l'invention réside dans la capacité de construire des promoteurs spécifiques des cellules sécrétrices des trichomes glanduleux, permettant une modification de la composition des sécrétions foliaires de la plante, et notamment d'y exprimer la terpène synthase permettant de préparer le terpène d'intérêt.

Par exemple, il a été montré qu'une séquence régulatrice de 1852 pb, située en amont de l'ATG du gène CYP71D16, permet de diriger l'expression du gène rapporteur *uidA* spécifiquement dans les cellules sécrétrices de trichomes de tabac (demande US2003/0100050 A1, Wagner et al., 2003). D'autre part, plusieurs séquences promotrices extraites de différentes espèces ont été identifiées comme étant capables de diriger l'expression d'un gène hétérologue dans les trichomes de tabac (Tableau 1).

Parmi ces promoteurs, celui du gène *LTP3,* codant pour une protéine du coton impliquée dans le transfert des lipides (LTP), s'exprime spécifiquement dans les cellules de fibres de coton. La séquence régulatrice du gène (1548 pb) a été étudiée chez le tabac. Cette séquence dirige de manière spécifique l'expression du gène *uidA* dans les trichomes de la feuille. La séquence de 315 pb localisée entre les positions -614 et -300 en amont de l'ATG serait responsable de la spécificité du promoteur. Le promoteur du gène *LTP6* permettrait également une expression spécifique des trichomes de coton. Sur la base des publications, il semblerait néanmoins que l'expression se localise dans les cellules de la base du trichome, et non dans les cellules sécrétrices. En outre, lorsque ces promoteurs sont introduits dans le tabac, l'expression n'est plus hautement spécifique, avec notamment un signal dans les cellules épidermiques (cf Tableau 1).

C'est pourquoi, dans un mode de réalisation préféré de la présente invention, le promoteur utilisé dans la cassette est dérivé des gènes NsTPS-02a, 02b, 03, et 04 de l'espèce *Nicotiana sylvestris* possédant une forte similarité de séquence avec CYC-2 (CBT-ol cyclase; NID : AF401234). La séquence de ces promoteurs est décrite dans les SEQ ID Nos 5-8. Ainsi, le promoteur contenu dans la cassette d'expression comprend un acide nucléique ayant une activité de promoteur transcriptionnel fonctionnel dans les trichomes glanduleux, **caractérisé en ce qu**'il comprend tout ou partie de la séquence SEQ ID NO: 5, 6, 7 ou 8 ou d'un variant fonctionnel de celles-ci. Notamment, par un variant fonctionnel de celles-ci est entendu une séquence présentant au moins 80, 85 ou 90% d'identité avec l'une d'entre-elles (obtenus par le programme d'alignement de séquences blastN (Altschul et al., 1990)), et est spécifique des trichomes glanduleux, notamment des cellules sécrétrices des trichomes glanduleux. Ces séquences promotrices sont plus amplement décrites dans la demande de brevet FR n° 04 10799 déposée le 13 Octobre 2004.

Parmi les séquences terminateurs, on peut citer le terminateur NOS (Bevan et al., 1983, Nucleic Acids Res. 11(2), 369-385), et le terminateur du gène d'histone (EPO 633 317).

Dans un mode de réalisation particulier, la cassette d'expression peut comprendre une séquence permettant d'augmenter l'expression (« enhancer »), par exemple certains éléments du promoteur CaMV35S et de gènes de l'octopine synthase (US 5 290 924). De préférence, les éléments activateurs du promoteur CaMV35S sont utilisés. Un exemple d'élément activateur est donné dans la séquence SEQ ID No 9.

L'acide nucléique transcrit code pour la terpène synthase capable de synthétiser le terpène d'intérêt.

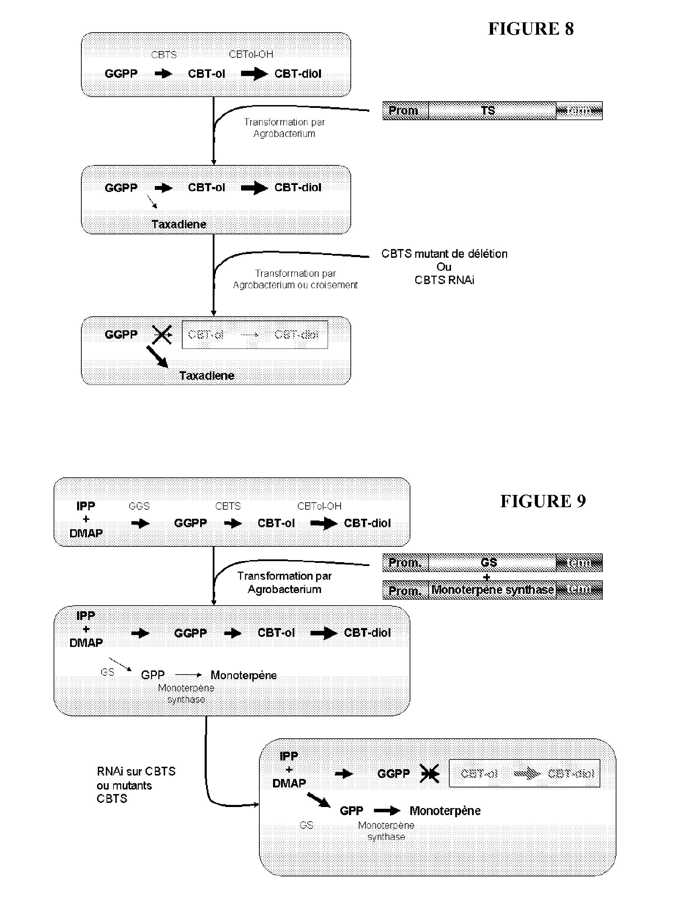

Dans un mode de réalisation particulier de l'invention, le terpène d'intérêt est un diterpène. De préférence, le diterpène d'intérêt est un taxane. Plus particulièrement, le taxane peut être la taxadiène. La terpène synthase hétérologue qui est introduite dans la plante est une diterpène syntase, plus particulièrement la diterpène synthase capable de synthétiser le diterpène d'intérêt. Par exemple, pour la taxadiène, la diterpène synthase est une taxadiène synthase. Plus particulièrement, la taxadiène synthase est celle d'if. La Figure 8 illustre la stratégie de production de taxadiène dans les trichomes de tabac. De nombreuses séquences codantes et séquences protéiques sont connues pour la taxadiène synthase d'if. A titre d'exemples non limitatifs, certaines références sont indiquées ci-dessous.

| Réf. Séquence codante | Réf. Séquence protéique |
|---|---|
| AY365032 | AAR15329.1 |
| AY364470 | AAR13861.1 |
| AY364469 | AA13860.1 |
| AY461450 | AAS18603.1 |
| U48796 | AAC49310.1 |

Par ailleurs, pour la casbène, la diterpène synthase est une casbène synthase. Plus particulièrement, la casbène synthase est celle de ricin. A titre d'exemple non limitatif, on peut citer la référence L32134 pour la séquence codante et la référence P59287 pour la séquence protéique.

Plus généralement, la présente invention couvre toute diterpène synthase connue ou dont la séquence répond aux caractéristiques de diterpènes synthases, comme indiqué ci-après.

Plusieurs diterpènes synthases ont vu leurs gènes clonés et leurs fonctions confirmées. Il s'agit de: la taxadiène synthase (Wildung and Croteau, 1996), l'abietadiène synthase (Peters *et al.,* 2000), la levopimaradiène synthase (Schepmann *et al.,* 2001), l'isopimaradiène synthase (Martin *et al.,* 2004), la casbène synthase (Mau et West, 1994), la cembratriène-ol synthase (Wang and Wagner, 2003), l'*ent*-cassadiène synthase (Cho *et al.,* 2004), la labdène synthase (Seo *et al.,* 2003), la *syn*-pimaradiène synthase (Wilderman *et al,.* 2004), l'ent-copalyl diphosphate synthase et l'ent-kaurène synthase (Sun *et al.,* 1994, Prisic *et al.,* 2004). D'autres gènes de diterpène synthases sont en cours de caractérisation chez le riz (la syn-stemarène synthase, la *syn*-pimaradiène synthase, la *ent*-sandaracopimaradiène synthase et la *ent*-cassadiène synthase) ou le tabac (*cis*-abienol synthase).

Les séquences protéiques des diterpènes synthases présentent des caractéristiques communes. Les protéines non-matures possèdent toutes à l'extrémité N-terminale un peptide d'adressage vers les plastes. Toutes les diterpènes synthases possèdent 2 domaines caractéristiques. Le domaine N-terminal porte le nom de *glycosyl hydrolase-like domain;* le domaine C-terminal contient le site catalytique.

Les diterpènes synthases sont subdivisées en 3 grandes classes. Les enzymes de la classe I possèdent un motif consensus DDXXD dans le domaine C-terminal. Les enzymes de la classe II possèdent un motif consensus (D/E)XD(D/N) qui est positionné dans le domaine N-terminal (Prisic *et al.,* 2004). Les enzymes des classes I et II agissent séquentiellement pour générer des squelettes carbonés de type labdanes et sont généralement caractéristiques du métabolisme primaire (ex.: *ent*-copalyl diphosphate synthase et *ent*-kaurène synthase), mais interviennent aussi dans le métabolisme secondaire comme chez le riz (ex.: *ent*-copalyl diphosphate synthase et *ent*-cassadiène synthase). D'autres enzymes spécifiques du métabolisme secondaires des gymnospermes, possèdent ces deux motifs et sont donc bi-fonctionnelles avec 2 sites catalytiques (ex.: abiétadiène synthase). Elles sont malgré leur bi-fonctionalité répertoriées dans la classe I. Toutes ces enzymes (classes I et II) sont caractérisées en plus par une séquence interne au domaine N-terminal appelée CDIS pour *Conifer Diterpene Internal Sequence.* Cette séquence est généralement d'environ 215 acide-aminés et est positionnée entre le peptide signal et le *glycosyl hydrolase-like domain* (Trapp et Croteau, 2001).

Chez les angiospermes, les enzymes de la classe III possèdent le consensus DDXXD en C-terminal, mais pas le CDIS des classes I et II. Ces enzymes agissent en une seule étape réactionnelle. La casbène synthase est un exemple de diterpène synthase de classe III.

Il existe dans le génome d'*Arabidopsis thaliana,* 21 séquences qui répondent aux critères des diterpènes synthases de classe III (Aubourg *et al.,* 2002). Leurs coordonnées sur le genome, selon la nomenclature internationale des genes d'Arabidopsis, sont les suivantes : At5g48110, At3g29410, At3g14490, At1g31950, At4g15870, At2g23230, At1g48800, At1g66020, At3g29110, At1g70080, At1g33750, At3g32030, At3g14520, At3g14540, At5g44630, At4g13280, At4g13300, At4g20210, At3g29190, At4g20230, At4g20200. Les protéines codées par les gènes At3g29410, At3g14540, At1g33750, At3g32030 et At1g48800, possèdent aussi un motif (D/E)EDD de type classe II dans le domaine N-terminal, ce qui les rapprochent des diterpènes synthases bi-fonctionelles de la classe I. Ces enzymes sont donc susceptibles de produire des diterpènes de type labdanoïdes en une seule étape. Aucune des protéines codées par ces séquences n'a été authentifiée. Toutefois, les inventeurs tentent de caractériser deux protéines codées par les gènes At3g29410, At3g14540.

Dans un autre mode de réalisation particulier de l'invention, le terpène d'intérêt est un monoterpène. Par exemple, le monoterpène d'intérêt peut être le limonène. A titre non limitatif, ce pourrait être également le carène, le pinène, le thujène, ou le linalool. La terpène synthase hétérologue qui est introduite dans la plante est une monoterpène synthase, plus particulièrement la monoterpène synthase capable de synthétiser le monoterpène d'intérêt. Cependant, la production de monoterpène nécessite en outre l'introduction d'une géranylpyrophosphate synthase exprimée dans les trichomes. A titre d'exemples non limitatifs, certaines références de géranylpyrophosphate synthase sont indiquées ci-dessous.

| Organismes | Réf. Séquence codante | Réf. Séquence protéique |
|---|---|---|
| Vitis vinifera | AY351862 | AAR08151 |
| Mentha piperata | AF182828 | AAF08793 |
| Abies grandis | AF513112 | AAN01134 |
| Arabidopsis t. | Y17376 | CAC16849 |

La Figure 9 illustre la stratégie de production de monoterpène dans les trichomes de tabac. Par exemple, pour le limonène, la monoterpène synthase est une limonène synthase. De nombreuses séquences codantes et séquences protéiques sont connues pour la limonène synthase. A titre d'exemples non limitatifs, certaines références sont indiquées ci-dessous.

| Réf. Séquence codante | Réf. Séquence protéique |
|---|---|
| AY473624 | AAS47694.1 |
| AF514289 | AAM53946.1 |
| AF514287 | AAM53944.1 |
| AF317695 | AAK06663.1 |
| AF241793 | AAG31438.1 |
| AF241792 | AAG31437.1 |
| AF241791 | AAG61436.1 |
| AF241790 | AAG31435.1 |
| AF233894 | AAF65545.1 |
| AF175323 | AAD50304.1 |

La carène synthase, la pinène synthase, la thujène synthase, et la linalool synthase sont d'autres exemple de monoterpènes synthases.

Dans un mode de réalisation particulier supplémentaire de l'invention, le terpène d'intérêt est un sesquiterpène. Par exemple, le sesquiterpène d'intérêt peut être le valencène, le santalène, le germacrène ou l'*epi*-aristolochène.

La terpène synthase hétérologue qui est introduite dans la plante est une sesquiterpène syntase, plus particulièrement la sesquiterpène synthase capable de synthétiser le sesquiterpène d'intérêt. Cependant, la production de sesquiterpène nécessite en outre l'introduction d'une farnésylpyrophosphate synthase exprimée dans les trichomes.

| Organismes | Réf. Séquence codante | Réf. Séquence protéique |
|---|---|---|
| Arabidopsis | L46367 | AAF44787 |
| Artemisia | AY308477 | AAP74720 |
| Mentha | AF384040 | AAK63847 |

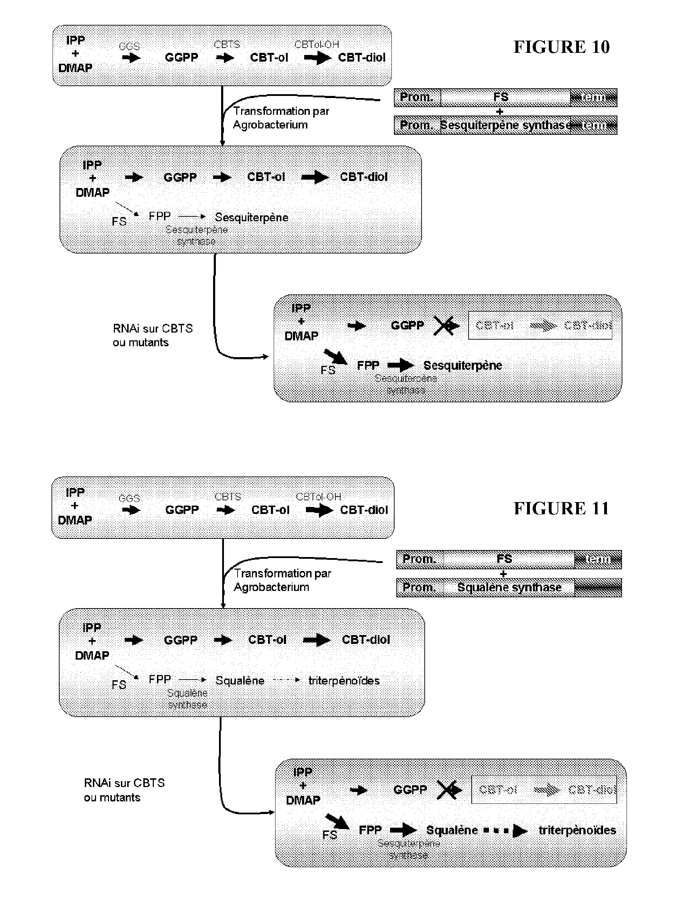

La Figure 10 illustre la stratégie de production de sesquiterpène dans les trichomes de tabac. Par exemple, pour le valencène, la sesquiterpène synthase est une valencène synthase. Plus particulièrement, la valencène synthase est celle d'orange douce. A titre d'exemple non limitatif, on peut citer la référence AF441124 pour la séquence codante et la référence AAG04608.1 pour la séquence protéique.

La germacrène synthase (gène SSTLH1, référence AF279455) et l'*épi*-aristolochène synthase (référence AAA19216) sont d'autres exemples de sesquiterpènes synthases.

Dans un mode de réalisation particulier additionnel de l'invention, le terpène d'intérêt est un triterpène. Par exemple, le triterpène d'intérêt peut être le lanosterol, le cycloartenol, le lupeol ou la beta-amyrin.

La terpène synthase hétérologue qui est introduite dans la plante est une triterpène syntase, plus particulièrement la triterpène synthase capable de synthétiser le triterpène d'intérêt. Cependant, la production de triterpène nécessite en outre l'introduction d'une farnésylpyrophosphate synthase, d'une squalène synthase, et d'une squalène époxydase exprimées dans les trichomes.

| Enzyme | Réf. Séquence codante | Réf. Séquence protéique |
|---|---|---|
| Squalene synthase | D29017 | BAA06103 |
| Squalene epoxidase | NM_104624 | NP_564734 |

La Figure 11 illustre la stratégie de production de triterpène dans les trichomes de tabac. Par exemple, pour le lanosterol, la triterpène synthase est une lanosterol synthase. De nombreuses séquences codantes et séquences protéiques sont connues pour la lanosterol synthase.

La lanosterol synthase, la cycloartenol synthase, la lupeol synthase et la beta-amyrin synthase sont d'autres exemples de triterpènes synthases.

Outre l'introduction d'une construction portant une cassette d'expression comprenant une séquence polynucléotidique codant une terpène synthase hétérologue, la méthode peut comprendre l'introduction dans la cellule de la plante d'un ou plusieurs transgènes codant chacun une enzyme de modification des terpènes. Notamment, la modification du terpène peut être une hydroxylation, une acylation et en particulier une acétylation, une benzoylation, une déshydrogénation, etc. L'introduction du transgène se fait de préférence par l'introduction d'une cassette d'expression comprenant une séquence polynucléotidique codant une enzyme de modification des terpènes. La séquence polynucléotidique codant une enzyme de modification des terpènes est sous le contrôle d'un promoteur permettant une expression dans les trichomes, de préférence spécifique des trichomes. Le transgène peut être porté par la construction comprenant la séquence polynucléotidique codant une terpène synthase hétérologue ou par une construction distincte. Par exemple, cette enzyme de modification peut être une monooxygénase à P450, une acyltransferase, une benzoyltransferase, une réductase, entre autres. Une liste non exhaustive de gènes codant des enzymes de modification de terpène est présentée dans le tableau ci-dessous.

| **Enzyme** | Gène (N° accession Genbank) | Squelette terpènique modifié |
|---|---|---|
| Taxoid 2-alpha hydroxylase | AY518383 | Taxadiene |
| Taxadiene 5-alpha hydroxylase | AY289209 | Taxadiene |
| Taxoid 7-beta hydroxylase | AY307951 | Taxadiene |
| Taxoid 10-beta hydroxylase | AY563635 | Taxadiene |
| 5-alpha-taxadienol-10-beta-hydroxylase | AY453403 | Taxadiene |
| Taxane 14b-hydroxylase | AY188177 | Taxadiene |
| Taxane 13-alpha-hydroxylase | AY056019 | Taxadiene |
| Taxane hydroxylase | AY374652 | Taxadiene |
| Taxadien-5-alpha-ol-O-acetyltransferase | AY628434 | Taxadiene |
| Taxadienol acetyl transferase | AF190130 | Taxadiene |
| 10-deacetylbaccatin III-10-O-acetyl transferase | AF193765 | Taxadiene |
| 2-debenzoyl-7,13-diacetylbaccatin III-2-O-benzoyl transferase | AF297618 | Taxadiene |
| 3'-N-debenzoyltaxol N-benzoyltransferase | AF297618 | Taxadiene |
| Taxoid phenylpropanoyltransferase | AY082804 | Taxadiene |
| taxane 2-alpha-O-benzoyltransferase | AY675557 | Taxadiene |
| Taxoid-O-acetyltransferase | AY628433 | Taxadiene |
| 5-epi-aristolochene-1,3-dihydroxylase | AF368376 | 5-epi-aristolochene |
| abietadienol/abietadienal oxidase | AY779538 | Abietadiene, dehydroabietadiene, levopimaradiene, isopimaradiene |
| Limonene-3-hydroxylase | AAQ18708 | Limonene |
| (-)-isopiperitenol dehydrogenase | AY641428 | Limonene |
| (-)-isopiperitenone reductase | AY300162 | Limonene |
| (+)-pulegone reductase | AY300163 | Limonene |
| menthol dehydrogenase | AY288138 | Limonene |

La cassette d'expression ainsi constituée est insérée dans un vecteur. Le vecteur peut être un ADN ou un ARN, circulaire ou non, simple- ou double-brin. Il s'agit typiquement d'un plasmide, phage, virus, cosmide, chromosome artificiel, etc. Il s'agit avantageusement d'un vecteur de plante, c'est-à-dire capable de transformer une cellule végétale. Des exemples de vecteurs de plantes sont décrits dans la littérature, parmi lesquels on peut citer notamment les plasmides T-DNA de *A. tumefaciens* pBIN19 (Bevan, 1984), pPZP100 (Hajdukewicz et al., 1994), série pCAMBIA (R. Jefferson, CAMBIA, Australie). Les vecteurs de l'invention peuvent comprendre, en outre, une origine de réplication et/ou un gène de sélection et/ou une séquence de recombinaison végétale, etc. Les vecteurs peuvent être construits par des techniques classiques de biologie moléculaire, bien connues de l'homme du métier, utilisant par exemple des enzymes de restriction, de ligature, des clonages, réplication, etc.

Les gènes de sélection comprennent, de manière non exclusive, l'utilisation de gènes marqueurs tels que des gènes conférant des résistances à un antibiotique ou à des herbicides, ou de systèmes de sélection positive, en particulier le système basé sur une sélection sur mannose, en présence du gène de sélection de la MPI (Mannose-6-phosphate isomérase) (Hansen et Wright, 1999), ou de systèmes de sélection couplés à l'élimination des gènes marqueurs après sélection (Ebinuma et al., 1997). Enfin, les plantes transformées peuvent également être sélectionnées par criblage PCR en l'absence de gènes marqueurs de sélection (McGarvey et Kaper, 1991).

L'introduction des constructions de l'invention dans une cellule ou un tissu végétal, y compris une graine ou plante, peut être réalisée par toute technique connue de l'homme du métier. Les techniques de transgenèse végétale sont bien connues dans le domaine, et comprennent par exemple l'utilisation de la bactérie *Agrobacterium tumefaciens,* l'électroporation, le transfert conjugatif, des techniques biolistiques, transfection par un vecteur viral notamment, et toute autre technique connue de l'homme du métier.

Une technique communément utilisée repose sur l'utilisation de la bactérie *Agrobacterium tumefaciens,* qui consiste essentiellement à introduire la construction d'intérêt (acide nucléique, cassette, vecteur, etc.) dans la bactérie *A. tumefaciens,* puis à mettre en contact cette bactérie transformée avec des disques de feuilles de la plante choisie. L'introduction de la cassette d'expression dans la bactérie est typiquement réalisée en utilisant comme vecteur le plasmide Ti (ou T-DNA), qui peut être transféré dans la bactérie par exemple par choc thermique. L'incubation de la bactérie transformée avec les disques foliaires permet d'obtenir le transfert du plasmide Ti dans le génome des cellules des disques. Ceux-ci peuvent éventuellement être cultivés dans des conditions appropriées pour reconstituer une plante transgénique dont les cellules comprennent la construction de l'invention. Pour plus de détails ou des variantes de mise en oeuvre de la technique de transformation par *A. tumefaciens,* on peut se référer par exemple à Horsch et al., 1985 ou Hooykaas and Schilperoort, 1992.

Ainsi, dans un mode de réalisation particulier, la cassette d'expression ainsi constituée est insérée entre les bordures gauche et droite de l'ADN de transfert (T-DNA) d'un plasmide Ti désarmé pour le transfert dans des cellules végétales par *Agrobacterium tumefaciens.* Le T-DNA comprend également un gène dont l'expression confère une résistance à un antibiotique et qui permet la sélection des transformants.

Une autre technique de transformation végétale est basée sur la projection de microparticules (typiquement des microbilles) auxquelles sont attachées les constructions génétiques, directement sur des cellules végétales, suivie de la culture de ces cellules pour reconstituer une plante transgénique. Les particules utilisées sont typiquement des particules d'or, qui sont projetées typiquement au moyen d'un canon à particules (voir notamment Russell et al., In Vitro Cell. Dev. Biol., 1992, 28P, p. 97-105).

La technique de microinjection repose essentiellement sur l'injection des constructions génétiques dans des embryons ou protoplastes végétaux, puis à cultiver ces tissus de manière à régénérer des plantes complètes. D'autres techniques de transgenèse végétale sont bien connues, ou d'autres protocoles mettant en oeuvre les techniques ci-dessus sont décrits dans l'art antérieur (Siemens, J and Schieder, 1996) et peuvent être appliqués à la présente invention.

La présente invention peut notamment être utilisée pour la production de terpènes d'intérêt spécifiquement dans les cellules sécrétrices de trichomes glanduleux des plantes supérieures (notamment les Angiospermes). L'invention est applicable notamment à toutes les plantes de familles possédant des trichomes glanduleux, par exemple les Astéracées (tournesol, etc...), Solanacées (tomate, tabac, pomme de terre, poivron, aubergine, etc...), Cannabacées (ex *Cannabis sativa*) et Lamiacées (menthe, basilic, lavande, thym, etc.). Elle est particulièrement adaptée aux plantes de la famille des Solanacées, telles que par exemple des genres Solanum, Lycopersicon, Capsicum, Petunia, Datura, Atropa, etc., et aux Nicotianées, par exemple le tabac, et plus particulièrement le tabac sauvage *Nicotiana sylvestris.* De manière non limitative, l'invention peut s'appliquer aux plantes des genres suivants : Populus, Lycopersicon, Nicotiana, Cannabis, Pharbitis, Apteria, Psychotria, Mercurialis, Chrysanthemum, Polypodium, Pelargonium, Mimulus, Matricaria, Monarda, Solanum, Achillea, Valeriana, Ocimum, Medicago, Aesculus, Plumbago, Pityrogramma, Phacelia, Avicennia, Tamarix, Frankenia, Limonium, Foeniculum, Thymus, Salvia, Kadsura, Beyeria, Humulus, Mentha, Artemisia, Nepta, Geraea, Pogostemon, Majorana, Cleome, Cnicus, Parthenium, Ricinocarpos, Hymennaea, Larrea, Primula, Phacelia, Dryopteris, Plectranthus, Cypripedium, Petunia, Datura, Mucuna, Ricinus, Hypericum, Myoporum, Acacia, Diplopeltis, Dodonaea, Halgania, Cyanostegia, Prostanthera, Anthocercis, Olearia, Viscaria.

Une fois régénérées, les plantes transgéniques peuvent être testées pour l'expression de la terpène synthase hétérologue ou la production du terpène d'intérêt dans les trichomes. Ceci peut être réalisé en récoltant l'exsudat des feuilles et en testant la présence du terpène d'intérêt dans cet exsudat, lorsque le terpène d'intérêt est destiné à être sécrété. Ceci peut également être fait en analysant la présence de la terpène synthase hétérologue dans les feuilles et, plus particulièrement, dans les cellules de trichome (par exemple en analysant les ARNm ou l'ADN génomique au moyen de sondes ou d'amorces spécifiques). Les plantes peuvent éventuellement être sélectionnées, croisées, traitées, etc. pour obtenir des plantes présentant des niveaux d'expression améliorés.

A cet égard, un autre objet de l'invention réside dans une cellule modifiée comprenant une cassette ou un vecteur tels que définis précédemment. Il peut s'agir par exemple d'une cellule de plante, notamment de la famille des Solanacées, Astéracées, Cannabacées ou Lamiacées. Les cellules peuvent être cultivées *in vitro,* et utilisées pour reconstituer des tissus ou plantes entières, pour produire des terpènes d'intérêt en culture, ou encore pour étudier les propriétés de terpènes synthases hétérologues (par exemple en génomique fonctionnelle).

Un autre objet de l'invention réside également dans une plante ou graine comprenant une cassette d'expression ou un vecteur tels que définis précédemment. Plus particulièrement, la présente invention concerne une plante ou graine transgènique présentant des trichomes glanduleux et comprenant une cassette d'expression contenant une séquence polynucléotidique codant pour une terpène synthase hétérologue permettant de synthétiser un terpène d'intérêt sous le contrôle d'un promoteur permettant une expression, de préférence spécifique, dans les trichomes. Lorsque la terpène synthase est une monoterpène synthase, la plante ou graine transgénique comprend en outre une cassette d'expression contenant une séquence polynucléotidique codant pour une géranylpyrophosphate synthase sous le contrôle d'un promoteur permettant son expression dans les trichomes. Lorsque la terpène synthase est une sesquiterpène synthase, la plante ou graine transgénique comprend en outre une cassette d'expression contenant une séquence polynucléotidique codant pour une farnésylpyrophosphate synthase sous le contrôle d'un promoteur permettant son expression dans les trichomes. Lorsque la terpène synthase est une triterpène synthase, la plante ou graine transgénique comprend en outre des cassettes d'expression contenant des séquences polynucléotidiques codant pour une farnésylpyrophosphate synthase, une squalène synthase et une squalène époxydase sous le contrôle de promoteurs permettant son expression dans les trichomes.

Dans un mode de réalisation préféré de la présente invention, la plante présente en outre une voie de production des terpènes endogènes bloquée dans les trichomes. Dans un mode de réalisation préféré, le blocage de la voie de production des terpènes endogènes est spécifique des trichomes, c'est-à-dire qu'elle est peu ou pas affectée dans les autres parties de la plante. Le blocage de la voie de production des terpènes endogènes est de préférence effectué en bloquant l'expression des terpènes synthases endogènes. Cependant, l'invention envisage également un blocage de la voie de production de terpènes endogènes à d'autre niveau.

L'expression des terpènes synthases endogènes peut être bloquée par de nombreuses techniques disponibles et connues par l'homme du métier. Les gènes des terpènes synthases peuvent être délétés, mutés (par ex., mutagenèse chimique par EMS ou rayonnements) ou interrompus (mutagenèse insertionnelle). Par ailleurs, le blocage de l'expression des terpènes synthases endogènes peut également être réalisé par extinction de gènes en exprimant un transcrit inhibiteur. Le transcrit inhibiteur est un ARN qui peut se présenter sous la forme d'un ARN double brin, d'un ARN antisens, d'un ribozyme, d'un ARN capable de former une triple hélice, et qui a une certaine complémentarité ou spécificité avec le transcrit de la diterpène endogène.

Selon un mode particulier de réalisation de la présente invention, le transcrit inhibiteur se présente sous la forme d'un ARN antisens. Ce dernier comprend généralement une séquence nucléotidique complémentaire d'au moins une partie du transcrit des terpènes synthases endogènes, et s'hybride de manière sélective à ces transcrits par des interactions de type Watson-Crick classique. Le ou les transcrits inhibiteurs de type ARN antisens peuvent donc se fixer aux transcrits des terpènes synthases et par exemple bloquer l'accès à la machinerie cellulaire de traduction à l'extrémité 5' du transcrit d'intérêt lorsque ce dernier est un ARNm, gêner sa traduction en protéine, et permettre la suppression de l'expression du transgène d'intérêt in vivo (Kumar et al., Microbiol. Mol. Biol. Rev, 62 (1993) 1415-1434). De tels polynucléotides ont été par exemple décrits dans les brevets EP 92574 et EP 140308. Lorsque le transcrit inhibiteur est de type ARN antisens, il peut couvrir toute ou partie de la séquence codante du transcrit de la diterpène synthase, ou toute ou partie de la séquence non codante en 3' ou en 5'. De préférence, le transcrit inhibiteur antisens est complémentaire de la séquence de fixation du ribosome et d'initiation de la traduction. De préférence, le transcrit inhibiteur a une longueur d'au moins 10 ribonucléotides.

Dans un mode de réalisation préféré, le transcrit inhibiteur met en jeu le mécanisme des ARN interférents (cf. revue de Baulcombe, 2004). De préférence, cette extinction est effectuée par la technique des *intron-spliced hairpin RNA* ou ihpRNA (Smith *et al.,* 2000). Elle consiste à produire un ARN double brin du ou des gènes ciblés via une construction comprenant un fragment sens et ce même fragment en orientation antisens, les deux étant séparés par un intron (Wesley et al., 2001 ; Wang et al., demande de brevet, 1999). Cette construction est de préférence sous contrôle d'un promoteur permettant une expression spécifique dans les trichomes.

Cependant, la présente invention considère également tout moyen connu de l'homme du métier pour bloquer la voie de production des terpènes endogènes dans les trichomes. En effet, il est important de préciser que le procédé d'extinction des gènes TPS peut faire l'objet d'autres approches. Par exemple, elle peut consister en la réalisation d'une collection de mutants de délétions par irradiation, par exemple par rayons gamma ou fast-neutrons. Les délétions affectant un locus donné peuvent être repérées par diverses méthodes sur l'ADN extrait des mutants (Tissier & Montané, 1999). Un avantage de la mutagenèse par rayonnement est la possibilité d'isoler des délétions couvrant la totalité d'un cluster de gènes. Ceci est particulièrement pertinent dans le cas des cembranes synthases de *Nicotiana,* puisque les gènes codant pour ces enzymes constituent une famille de gènes très similaires et clustérisés sur un locus (Tissier et al., 2004 ; Sallaud et coll., données non publiées).

La présente invention concerne également une plante ou graine transgénique selon la présente invention comprenant en outre un transgène codant pour une enzyme de modification des terpènes.

L'invention concerne en particulier une plante dont la voie de synthèse des diterpènes endogènes est bloquée dans les trichomes. Cette plante représente un intermédiaire important pour la préparation de la plante finale capable de produire le terpène d'intérêt. En effet, cette plante peut être obtenue par croisement d'une plante capable de produire le terpène d'intérêt avec une plante dont la voie de synthèse des diterpènes endogènes est bloquée dans les trichomes. L'invention concerne donc tout particulièrement une plante dont la voie de synthèse des diterpènes endogènes est bloquée dans les trichomes. Elle concerne en outre l'utilisation d'une plante transgénique dont la voie de synthèse des diterpènes endogènes est bloquée dans les trichomes pour la préparation d'une plante ou d'une graine transgénique comprenant une cassette d'expression contenant une séquence polynucléotidique codant pour une terpène synthase hétérologue permettant de synthétiser un terpène d'intérêt sous le contrôle d'un promoteur permettant une expression dans les trichomes. Elle concerne en outre une méthode de préparation d'une plante ou d'une graine transgénique dont la voie de synthèse des diterpènes endogènes est bloquée dans les trichomes et qui est capable de produire un terpène d'intérêt comprenant le croisement d'une plante transgénique dont la voie de synthèse des diterpènes endogènes est bloquée dans les trichomes avec une plante transgénique comprenant une cassette d'expression contenant une séquence polynucléotidique codant pour une terpène synthase hétérologue permettant de synthétiser un terpène d'intérêt sous le contrôle d'un promoteur permettant une expression dans les trichomes.

L'invention a encore pour objet un procédé d'obtention de plantes transformées **caractérisé en ce qu**'il comprend les étapes suivantes : a) obtention d'une cellule hôte recombinante végétale comprenant une cassette d'expression selon l'invention ; b) régénération d'une plante entière à partir de la cellule hôte recombinante obtenue à l'étape a) ; c) sélection des plantes obtenues à l'étape b) ayant intégré une cassette d'expression telle que définie dans la présente description.

L'invention a encore pour objet un procédé d'obtention d'une plante transformée **caractérisé en ce qu**'il comprend les étapes suivantes : a) obtention d'une cellule hôte recombinante d'*Agrobacterium tumefaciens* selon l'invention ; b) transformation d'une plante d'intérêt par infection avec les cellules hôtes recombinantes d'*Agrobacterium tumefaciens* obtenues à l'étape a) ; c) sélection des plantes ayant intégré dans leur génome une cassette d'expression telle que définie dans la présente description.

L'invention a encore pour objet un procédé d'obtention d'une plante transformée **caractérisé en ce qu**'il comporte les étapes suivantes : a) transfecter au moins une cellule de plante avec une cassette d'expression ou avec un vecteur recombinant selon l'invention ; b) régénérer une plante entière à partir de la cellule de plante recombinante obtenue à l'étape a) ; c) sélectionner les plantes ayant intégré dans leur génome une cassette d'expression selon l'invention.

L'un quelconque des procédés d'obtention d'une plante transformée décrit ci-dessus peut en outre comporter les étapes additionnelles suivantes : d) croisement entre elles de deux plantes transformées telles qu'obtenues à l'étape c) avec une plante de la même espèce ; e) sélection des plantes homozygotes pour le transgène.

Dans un second mode de réalisation particulier, l'un quelconque des procédés d'obtention d'une plante transgénique décrit ci-dessus peut en outre comporter les étapes additionnelles suivantes : f) croisement d'une plante transformée obtenue à l'étape c) avec une plante de la même espèce ; g) sélection des plantes issues du croisement de l'étape f) ayant conservé le transgène.

Les plantes transgèniques hybrides, obtenues par le croisement d'au moins une plante selon l'invention avec une autre, font également partie de l'invention.

Enfin, la présente invention concerne une méthode de récolte de terpènes hétérologues ou d'intérêt dans l'exsudat des trichomes d'une plante, comprenant a) la récolte de parties aériennes de la plante ; b) l'incubation de ces parties aériennes dans un solvant de type peu polaire ou apolaire ; et c) l'élimination du solvant. De préférence, ladite plante est une plante transgénique selon la présente invention et notamment le tabac. Par parties aériennes sont entendues de préférence les feuilles et les tiges. Le solvant peu polaire peut être le chlorure de méthylène ou le chloroforme. Dans un mode de réalisation particulier, le solvant est apolaire, de préférence très apolaire. Par exemple, le solvant peut être le pentane ou l'hexane ou tout solvant présentant la même polarité, de préférence le pentane. L'étape d'incubation peut durer de quelques secondes sous agitation à plusieurs heures dans un bain non agité. De préférence, le solvant choisi est volatile à température ambiante et présente une totale innocuité chimique à l'égard des terpènes d'intérêt. De préférence, l'élimination du solvant est effectuée par évaporation de celui-ci. Cependant, tout technique permettant d'éliminer le solvant est considérée dans la présente invention.

Par ailleurs, les inventeurs ont découvert que les plantes, dans lesquelles la production d'un composé ayant des propriétés antibiotiques à la surface des feuilles est bloquée, présentent une efficacité augmentée de transformation par une bactérie permettant le transfert d'ADN dans des cellules végétales. Le composé peut avoir des propriétés antibactériennes. Dans un mode de réalisation préféré, le blocage de la production du composé est spécifique des trichomes.

En effet, les terpènes, notamment ceux qui sont sécrétés à la surface des feuilles, sont des composés qui présentent souvent une activité antibiotique (voir par exemple les références : Trombetta *et al.,* 2005 ; Chorianopoulos *et al.,* 2004 ; Friedman et al., 2004 ; Rios & Recio, 2005 ; Saroglou *et al.,* 2005). Par conséquent, la présence de ces terpènes ayant une activité antibiotique constitue un obstacle à la transformation de ces plantes par des bactéries qui sont utilisées à ces fins, notamment des genres Agrobacterium, Rhizobium, Mesorhizobium ou Sinorhizobium (Broothaerts *et al.,* 2005). En effet, ces molécules auront une action inhibitrice sur la prolifération des bactéries lors de la transformation et inhiberont ainsi le transfert d'ADN vers les cellules végétales. Par conséquent, l'élimination de ces terpènes ayant une activité antibiotique pourrait soit rendre possible la transformation d'espèces récalcitrantes, soit augmenter les fréquences de transformation sur des espèces difficilement transformables.

De préférence, cette bactérie appartient aux genres Agrobacterium, en particulier *Agrobacterium tumefaciens,* Rhizobium, Sinorhizobium ou Mesorhizobium.

Le composé ayant des propriétés antibiotiques peut être un terpène. Par exemple, il peut être un diterpène. Dans un mode de réalisation préféré, le composé est le CBT-diol. Dans ce cas, la production de ce terpène peut être bloquée en bloquant l'expression des terpènes synthases endogènes dans les trichomes, en particulier d'une diterpène synthase, de préférence de la cembratriène-ol synthase. Les moyens disponibles pour réaliser ce blocage ont été décrits en détail plus haut. Notamment, on peut citer la mutagenèse physico-chimique du gène, la délétion du gène, une mutation insertionnelle de celui-ci ou une méthode de « gene silencing ». Cette dernière méthode est préférée.

De façon alternative, le composé ayant des propriétés antibiotiques peut être, et ce de manière non-exhaustive un des composes suivants: α-pinene, myrcene, ocymene, α-terpinene, p-cymene, carvacrol, thymol, linalool, camphor, terpineol, β-caryophyllene, caryophyllene oxide, patchoulol, germacrenes (A, B, C ou D), β-selinene, cadinene, bisabolenes (α, β, y), bisabolol, santalenes (α et β), santalols, etc., mais également les sesquiterpènes lactones présents dans de nombreuses espèces d'Asteracées.

La présente invention concerne donc l'utilisation de telle plante transgénique pour transformer cette plante avec une bactérie permettant le transfert d'ADN dans des cellules végétales. Elle concerne en outre une méthode pour transformer une plante, ladite plante présentant un blocage de la production d'un composé ayant des propriétés antibiotiques à la surface des feuilles, comprenant la mise en contact d'une bactérie permettant le transfert d'ADN dans des cellules végétales et portant un transgène avec une cellule de ladite plante. De préférence, la cellule de ladite plante est comprise dans un fragment de feuille, en particulier un disque foliaire. L'invention a encore pour objet un procédé d'obtention de plantes transformées **caractérisé en ce qu**'il comprend les étapes suivantes : a) obtention d'une cellule hôte recombinante d'une bactérie permettant le transfert d'ADN dans des cellules végétales comprenant un transgène, de préférence *Agrobacterium tumefaciens;* b) transformation d'une plante présentant un blocage de la production d'un composé ayant des propriétés antibiotiques à la surface des feuilles par infection avec les cellules hôtes recombinantes de bactéries obtenues à l'étape a) ; c) sélection des plantes ayant intégré dans leur génome le transgène. Le procédé d'obtention d'une plante transformée décrit ci-dessus peut en outre comporter les étapes additionnelles suivantes : d) croisement entre elles de deux plantes transformées telles qu'obtenues à l'étape c) avec une plante de la même espèce ; e) sélection des plantes homozygotes pour le transgène. En outre, le procédé peut comprendre les étapes additionnelles suivantes : f) croisement d'une plante transformée obtenue à l'étape c) avec une plante de la même espèce ; g) sélection des plantes issues du croisement de l'étape f) ayant conservé le transgène.

La présente invention concerne l'utilisation d'une plante, dans laquelle la voie de production de diterpène endogène, en particulier de CBT-diol, est bloquée dans les trichomes, pour identifier la fonction de gènes de biosynthèse de terpènoïdes. Plus particulièrement, le blocage de la voie de production de CBT-diol peut être effectué en bloquant l'expression de la cembratriène-ol synthase.

D'autres aspects et avantages de la présente invention apparaîtront à la lecture des exemples qui suivent, qui doivent être considérés comme illustratifs et non limitatifs.

### DESCRIPTION DES FIGURES

**Figure 1** **:** Cassette d'expression non spécifique de la taxadiène synthase dans les cellules de tabac.
**Figure 2** **:** Cassette d'expression dans les trichomes de tabac de la taxadiène synthase.
**Figure 3** **:** RNAi construction pour l'extinction des gènes *NsTPS.*
**Figure 4** **:** Cassette d'expression dans les trichomes de tabac de la casbène synthase.

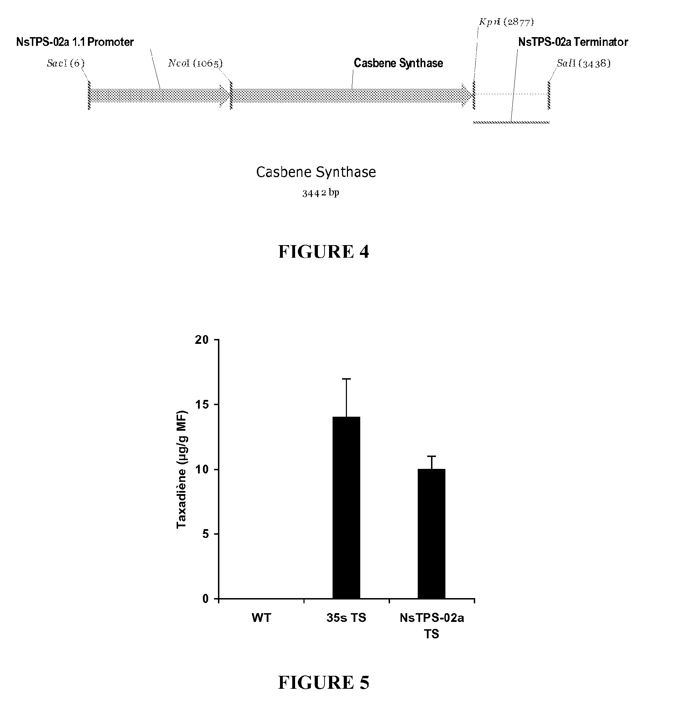

**Figure 5** **:** Sécrétion de la taxadiène dans les lignées exprimant la taxadiène synthase sous le contrôle de promoteurs 35S ou *NsTPS-02a.* Les constructions avec le promoteur 35S ou *NsTPS-02a* contrôlant l'expression de la taxadiène synthase, ont été introduites dans le génome de *Nicotiana sylvestris.* La taxadiène sécrétée dans l'exsudat de plantes contenant une seule copie du transgène a été extraite avec du pentane et quantifiée par analyses GC-MS. La quantité de taxadiène est exprimée en µg/g de matière fraîche. Le nombre de plantes analysées est N=12 et 5 pour les constructions avec le promoteur 35S et *NsTPS-02a* respectivement. (TS: Taxadiène Synthase, WT: Wild Type)
**Figure 6** **:** Quantité de CBT-diol sécrétée dans les plantes exprimant le RNAi ihpTPS. Le CBT-diol a été extrait de l'exsudat de *Nicotiana sylvestris* en utilisant le pentane, et quantifié par GC-MS. La quantité de CBT-diol est associée à 100% pour le WT. Les plantes (T1) issue de la descendance des transformants, sont représentées par ihp*TPS.*
**Figure 7** : Effet d'éléments activateurs du promoteur 35S sur l'expression dans les trichomes. Les ARN totaux de feuilles de tabac (*N. Sylvestris)* sont extraits et convertis en ADN complémentaires par transcription reverse. Le rapport d'expression est mesuré par PCR quantitative réalisée en duplex (fluorophore VIC pour le CYP71D16 et FAM pour le transgène). Le nombre de plante analysées est N=5. (e35S: éléments activateurs du promoteur 35S, p: promoteur).
**Figure 8** : Schéma récapitulant les étapes conduisant à la production de taxadiène dans les trichomes de tabac *(Nicotiana sylvestris).* GGPP : géranylgéranyl pyrophosphate ; CBT-ol : cembratriène-ol ; CBT-diol : cembratriène-diol ; CBTS : cembratriène-ol synthase ; CBToI-OH : cembratriène-ol hydroxylase. Prom : désigne un promoteur qui permet l'expression dans les trichomes, de préférence de manière spécifique. TS : taxadiène synthase ; term : élément permettant la terminaison de la transcription. CBTS RNAi : désigne une plante de tabac dans laquelle les gènes permettant la synthèse de CBTS sont éteints par une construction de type ihpRNA (cf exemple).
**Figure 9** : schéma récapitulant les étapes conduisant à la production de monoterpène dans les trichomes de tabac *(Nicotiana sylves tris).* Légende : GGS : géranylgéranylpyrophosphate synthase ; GS : géranylpyrophosphate synthase et idem Figure 7.
**Figure 10** : schéma récapitulant les étapes conduisant à la production de sesquiterpène dans les trichomes de tabac *(Nicotiana sylves tris).* Légende : FS : farnésylpyrophosphate synthase et idem Figures 7 et 8.
**Figure 11** **:** schéma récapitulant les étapes conduisant à la production de triterpènes dans les trichomes de tabac (*Nicotiana sylvestris*).
**Figure 12** **:** Illustration de l'augmentation de l'efficacité de transformation génétique obtenue à partir de disques foliaires de feuilles de plantes *N. sylvestris* dont la production en CBT-diol a été fortement réduite. La photo a été prise 3 semaines après co-culture avec la même souche d'*Agrobactérium tumefaciens* contenant un gène de résistance à la kanamycine et un transgène d'intérêt. WT : *N. sylvestris* ; *ihpTPS : N. sylvestris* contenant le transgène ihpTPS.

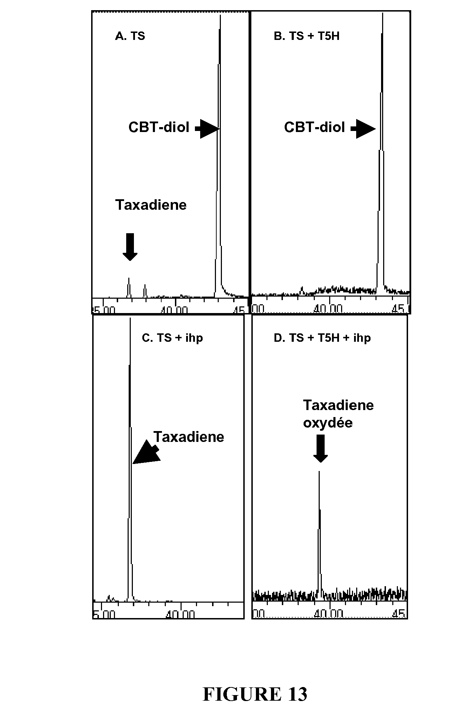

**Figure 13. Fig. 13A.** TS : Chromatogramme d'exsudat de plantes exprimant la taxadiène synthase seule. La taxadiène est détectable dans l'exsudat par GC/MS en extrayant l'ion 122, caractéristique de la taxadiène. **Fig. 13B.** TS + T5H : Chromatogramme GC-MS (ion extrait 191) d'exsudat de plantes exprimant la taxadiène synthase (TS) et la taxadiène 5-hydroxylase (T5H). La taxadiène n'est plus visible, et aucun autre produit n'est visible, sinon le CBT-diol. **Fig. 13C.** TS + ihp*TPS.* Chromatogramme GC-MS (ion extrait 122) d'exsudat de plantes exprimant la TS dans un contexte ihpTPS. Le CBT-diol est éliminé, facilitant la détection de taxadiène. **Fig. 13D.** TS+T5H+ihp*TPS*. Chromatogramme GC-MS (ion extrait 191) d'exsudat de plantes exprimant la TS et la T5H dans un contexte ihp*TPS*. Le CBT-diol n'est plus détectable, mais un pic correspondant à une taxadiène oxydée (produit de l'action de la taxadiène 5-hydroxylase sur la taxadiène) est facilement détectable.

### EXEMPLES

### Production de taxadiène dans le plant de tabac Nicotiana sylvestris par expression de la taxadiène synthase sous le contrôle d'un promoteur non spécifique.

Les constructions décrites ci-dessous ont permis une expression dans l'ensemble des cellules de la plante, y compris dans les trichomes. Cette expression est qualifiée de non spécifique des trichomes.

### Vecteur d'expression

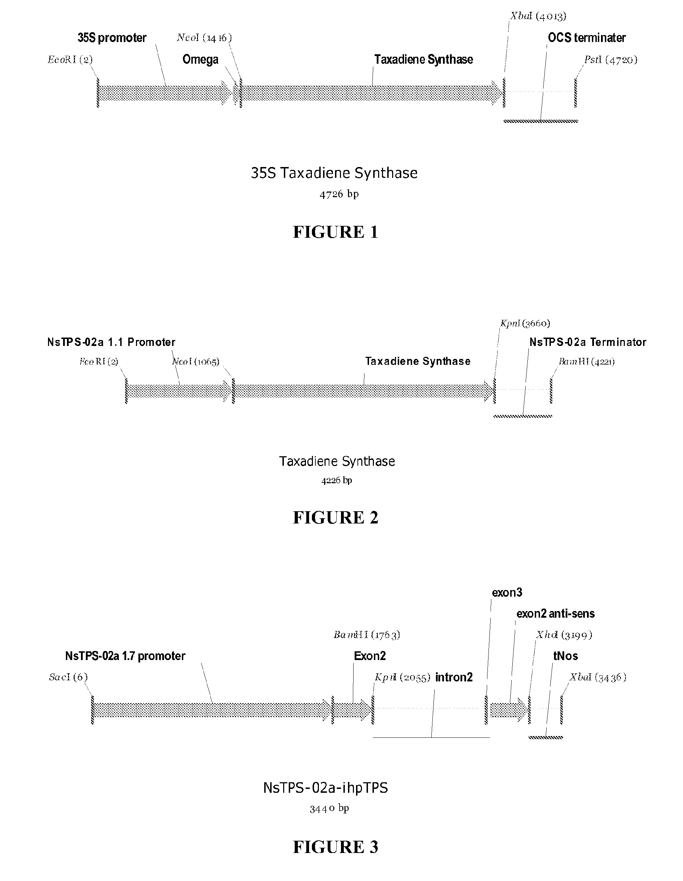

La construction de la cassette d'expression a été comme suit (Figure 1 : schéma) :
Un promoteur constitutif de type 35S (extrait du virus de la mosaïque du Chou-fleur) bien connu de l'homme du métier.
L'ADNc de la taxadiène synthase.
Le terminateur OCS (extrait du gène codant pour l'octopine synthase d'un plasmide Ti *d'Agrobacterium tumefaciens*) bien connu de l'homme du métier.
La séquence de cette construction est décrite dans la séquence SEQ ID No 1.

### Analyse des plantes transformées

Le processus de régénération a conduit à isoler des plantes transgéniques exprimant la taxadiène synthase. Les plantes contenant une seule copie du transgène ont été sélectionnées et soumises à l'analyse par chromatographie gazeuse couplée à une détection par spectrométrie de masse (GC-MS). L'exsudat de ces plantes contenait une quantité de 14 ± 3 µg/g de MF (matière fraîche) (Figure 5). La croissance de ces plantes était retardée par rapport au contrôle sauvage (non transformé) rendant celle-ci peu adaptée à la culture. Ces effets ont déjà été observés lors de la surexpression de diterpènes synthases chez la tomate ou *Arabidopsis thaliana* et ont été attribués à la diminution du pool de GGPP disponible dans les plantes transgéniques (Fray *et al.,* 1995 ; Besumbes *et al.,* 2004). Le GGPP est un métabolite important qui sert à la synthèse d'hormones telles que les gibbérellines et l'acide abscissique.

### Production de taxadiène spécifiquement dans les trichomes de tabac Nicotiana sylvestris.

Dans cet exemple, la taxadiène synthase a été placée sous contrôle du promoteur *NsCBTS-02a* spécifique des trichomes (Tissier et coll., 2004 ; Brevet n° FR 0410799) afin de restreindre la production de taxadiène aux trichomes.

### Vecteur d'expression

La cassette d'expression a été comme suit (Figure 2 : schéma):
Le promoteur *NsCBTS-02a* d'une taille de 1 kilobase.
L'ADNc de la taxadiène synthase.
Le terminateur du gène *NsCBTS-02a.*
La séquence de cette construction est décrite dans la séquence SEQ ID No 2.

### Analyse des plantes transformées

Le processus de régénération a conduit à régénérer des plants de tabac transgénique exprimant la taxadiène synthase spécifiquement dans les cellules sécrétrices de trichomes. Les plantes contenant une seule copie du transgène ont été sélectionnées. L'analyse GC-MS de l'exsudat a révèlé une teneur en taxadiène similaire à celle mesurée avec l'expression non-spécifique de la taxadiène synthase sous le promoteur 35S (10 ± 1 µg/g de MF, Fig. 5). La croissance de ces plantes était identique à celle des plantes sauvages (non transformées). De plus, les fleurs ont été normalement fertiles. Ceci démontre que la synthèse spécifique de la taxadiène dans les trichomes ne provoque aucun effet délétère pour la plante, et donc la supériorité de l'expression spécifique des trichomes par rapport à l'expression non-spécifique.

### Augmentation de la production de taxadiène par les trichomes de Nicotiana sylvestris en inhibant l'expression des gènes NsCBTS

L'inactivation des gènes *NsCBTS* a été réalisée par extinction des gènes, qui mettait en jeu le mécanisme des ARN interférents (cf. revue de Baulcombe, 2004). Cette extinction a été effectuée par la technique des *intron-spliced hairpin RNA* ou ihpRNA (Smith *et al.,* 2000). Elle consiste à produire un ARN double brin du ou des gènes ciblés via une construction comprenant un fragment sens et ce même fragment en orientation antisens, les deux étant séparés par un intron (Wesley et al., 2001 ; Wang et al., demande de brevet 1999). Plus précisément la cassette a été organisée comme suit (Figure 3):
Un fragment du promoteur *NsCBTS-02a* de 1,7 kb (brevet n° FR 0410799).
Un fragment comprenant l'exon 2 et l'intron 2 du gène *NsCBTS-02a* suivis de l'exon 2 de ce même gène en orientation anti-sens.
Le terminateur NOS.

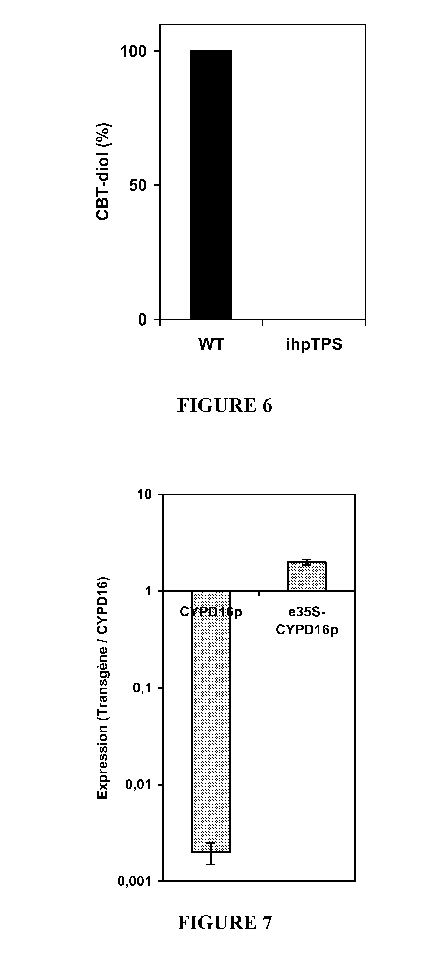

La transformation de cette cassette par *Agrobacterium tumefaciens* a permis d'obtenir des plantes transgéniques exprimant la construction ihpRNA pour les gènes *CBTS.* Les plantes contenant une seule copie du transgène ont été sélectionnées. La descendance de ces plantes a été analysée pour déterminer la quantité de CBT-diol. La Figure 6 indique que la sécrétion de CBT-diol est presque complètement interrompue dans les plantes *ihpTPS* (0,1 à 1 % du WT).

Ces plantes ont ensuite été croisées aux plantes exprimant la taxadiène synthase spécifiquement dans les trichomes (cf ci-dessus). Dans la descendance du croisement, les plantes portant les deux constructions ont été sélectionnées sur milieux sélectifs et analysées pour leur contenu en taxadiène par GC-MS.

La production de taxadiène dans ces plantes est environ 30 fois supérieure à celle des plantes exprimant seulement la cassette d'expression de la taxadiène sous le contrôle du promoteur trichome spécifique.

### Production de casbène spécifiquement dans les trichomes de tabac Nicotiana

### sylvestris.

### Vecteur d'expression

L'expression spécifique de la casbène synthase dans les trichomes a nécessité l'utilisation du promoteur spécifique *NsCBTS-02a* dans une cassette d'expression comme suit (Figure 4 : schéma et séquence) :
Le promoteur *NsCBTS-02a* de 1 kilobase.
L'ADNc complet de la casbène synthase.
Le terminateur du gène *NsCBTS-02a.*

### Analyse des plantes transformées

Le processus de régénérations a conduit à isoler des plantes transgéniques exprimant la casbène synthase spécifiquement dans les cellules de trichomes. L'analyse GC-MS des plantes contenant une seule copie du transgène a révèlé une teneur en casbène de l'ordre de 15 µg/g de MF. La croissance de ces plantes était identique à celle des plantes sauvages (non transformée). Ceci confirme, comme pour la taxadiène, que la synthèse spécifique de la casbène synthase dans les trichomes ne provoque aucun effet délétère pour la plante.

### Augmentation de l'expression d'un transgène par utilisation d'un activateur (enhancer) de transcription 35S

L'expression de la taxadiène synthase sous le contrôle du promoteur 1.8 kb du gène CYP71D16 a été comparée avec l'expression du gène codant pour taxadiène-5a-hydroxylase sous contrôle du même promoteur mais précédé par un élément activateur du promoteur 35S (SEQ ID No 9). L'analyse quantitative de l'expression a été réalisée par PCR quantitative en temps réel et avec l'utilisation de sondes TaqMan® spécifiques des gènes.

Les résultats présentés figure 7 indiquent que l'expression du gène taxadiène-5α-hydroxylase est 1000 fois supérieur à l'expression du gène de la taxadiène synthase. Nous pouvons donc en déduire que les éléments activateurs du promoteur 35S sont à l'origine de cette activation puisque les promoteurs sont par ailleurs identiques.

### Augmentation de l'efficacité de transformation génétique par Agrobacterium tumefaciens.

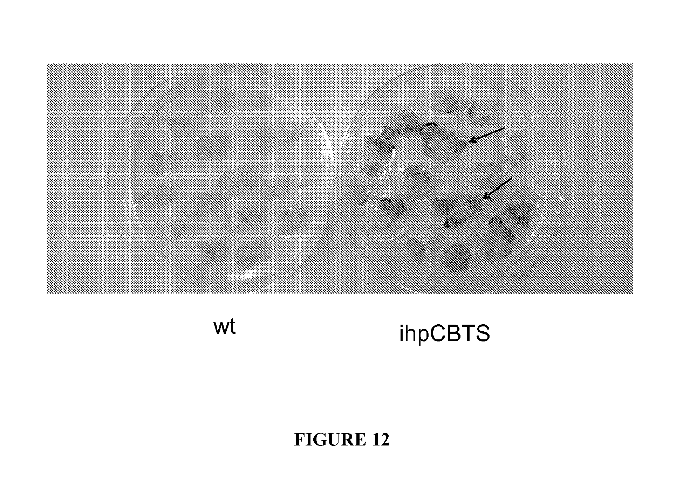

Une lignée homozygote portant le transgène ihpTPS (lignée #804) a été produite à partir de *Nicotiana sylvestris.* Cette lignée ne produit plus de CBT-diol à la surface des feuilles (cf. Figure 6). Des disques foliaires obtenus à partir de feuilles de *N. sylvestris* et de la lignée #804 ont été infectés par différentes souches d'*Agrobacterium tumefaciens* contenant un T-DNA portant un gène de résistance à la kanamycine et différents transgènes d'intérêts (Tableau 2). Après 3 jours de co-culture avec *Agrobacterium tumefaciens,* les disques foliaires ont été transférés sur un milieu sélectif contenant de la kanamycine (150 mg/L). Après 4 semaines de sélection, un plus grand nombre de cals résistants apparaît sur les disques de la lignée #804 (Fig. 12). Au final, le nombre de plantes transformées obtenues avec la lignée #804 est 5 à 10 fois supérieur par rapport au contrôle *N. sylvestris.* Ces résultats ont été confirmés par plusieurs expériences utilisant des souches d'*Agrobacterium* portant différents transgènes (Tableau 2). En conclusion, l'élimination du CBT-diol excrété à la surface de la feuille permet d'améliorer l'efficacité de transformation génétique.

### Identification de la fonction d'un gène codant pour une monooxygénase à cytochrome P450 d'if.

Un transgène codant pour la taxadiene synthase (NID : U48796) sous la dépendance d'un promoteur trichome spécifique (Demande de brevet n° FR 0410799) et un transgène codant pour une taxadiene 5-hydroxylase (T5H, NID : AY289209) sous la dépendance du même promoteur ont été introduits par d'*Agrobacterium tumefaciens* dans *N. sylvestris* et dans la lignée *ihpTPS* (#804). L'exsudat des feuilles des plantes transformées a été extrait par immersion des feuilles dans du pentane. L'analyse des composés présents dans l'exsudat a été réalisée par chromatographie en phase gazeuse suivie d'une détection par spectrométrie de masse (GC/MS). Les plantes possédant uniquement le transgène taxadiene synthase produisent de la taxadiene dans les exsudats (Fig. 13A). Les plantes possédant les deux transgènes ne produisent plus de taxadiene confirmant que la taxadiene est substrat de la T5H. Dans les plantes *ihpTPS,* un nouveau produit majoritaire apparaît de manière très visible sur le chromatogramme GC/MS (Fig. 13D). Dû à l'abondance de CBT-diol dans les exsudats des plantes *N*. *sylvestris,* ce produit n'est pas détectable dans ces plantes possédant également les deux transgènes (Fig. 13B). En conclusion, le très faible taux en CBT-diol dans l'exsudat des lignées ihp*TPS,* permet d'identifier plus facilement le produit d'une enzyme exprimée dans les trichomes et donc de déterminer la fonction de gènes correspondants. De plus, le produit est également facilement purifiable, permettant ainsi sa caractérisation et sa production.

### REFERENCES

Altschul SF, Gish W, Miller W, Myers EW, Lipman, DJ (1990) J. Mol. Biol. 215:403-410
Aubourg S, Lecharny A, Bohlmann J (2002), Genomic analysis of the terpenoid synthase (AtTPS) gene family of Arabidopsis thaliana. Mol. Genet. Genomics. 267: 730-745.
Bohlmann J, Meyer-Gauen G, Croteau R (1998) Plant terpenoid synthases: molecular biology and phylogenetic analysis. Proc Natl Acad Sci U S A. 95:4126-33.
Baulcombe D (2004) RNA silencing in plants. Nature 431:356-363.
Besumbes O, Sauret-Gueto S, Phillips MA, Imperial S, Rodriguez-Concepcion M, Boronat A (2004) Metabolic engineering of isoprenoid biosynthesis in Arabidopsis for the production of taxadiene, the first committed precursor of Taxol. Biotechnol. Bioeng. 88:168-75.
Botella-Pavia P, Besumbes O, Phillips MA, Carretero-Paulet L, Boronat A, Rodriguez-Concepcion M (2004) Regulation of carotenoid biosynthesis in plants: evidence for a key role of hydroxymethylbutenyl diphosphate reductase in controlling the supply of plastidial isoprenoid precursors. Plant J. 40:188-99.
Broothaerts W, Mitchell HJ, Weir B, Kaines S, Smith LM, Yang W, Mayer JE, Roa-Rodriguez C, Jefferson RA (2005). Gene transfer to plants by diverse species of bacteria. Nature. 433:629-633.
Cho EM, Okada A, Kenmoku H, Otomo K, Toyomasu T, Mitsuhashi W, Sassa T, Yajima A, Yabuta G, Mori K, Oikawa H, Toshima H, Shibuya N, Nojiri H, Omori T, Nishiyama M, Yamane H (2004) Molecular cloning and characterization of a cDNA encoding ent-cassa-12,15-diene synthase, a putative diterpenoid phytoalexin biosynthetic enzyme, from suspension-cultured rice cells treated with a chitin elicitor. Plant J. 37:1-8.
Chorianopoulos N, Kalpoutzakis E, Aligiannis N, Mitaku S, Nychas GJ, Haroutounian SA (2004). Essential oils of Satureja, Origanum, and Thymus species: chemical composition and antibacterial activities against foodborne pathogens. J Agric Food Chem. 52:8261-8267.
Ebinuma H, Sugita K, Matsunaga E, Yamakado M (1997) Selection of marker-free transgenic plants using the isopentenyl transferase gene. Ploc Natl Acad Sci U S A. Mar 18;94(6):2117-2121.
Fray RG, Wallace A, Fraser PD, Valero D, Hedden P, Bramley PM, Grierson D (1995) Constitutive expression of a fruit phytoene synthase gene in transgenic tomatoes causes dwarfism by redirecting metabolites from the gibberellin pathway. Plant J. 8:693-701.
Friedman M, Henika PR, Levin CE, Mandrell RE (2004). Antibacterial activities of plant essential oils and their components against Escherichia coli O157:H7 and Salmonella enterica in apple juice. J Agric Food Chem. 52:6042-6048.
Hansen G, Wright MS (1999) Recent advances in the transformation of plants. Trends Plant Sci. 4: 226-231.
Heemann V, Brümmer U, Paulsen C, Seehofer F (1983) Composition of the leaf surface gum of some Nicotiana species and Nicotiana tabacum cultivars. Phytochem. 22:133-135.
Hooykaas PJ, Schilperoort RA (1992) Agrobacterium and plant genetic engineering. Plant Mol Biol 20: 175
Horsch RB, Rogers SG, Fraley RT (1985) Transgenic plants. Cold Spring Harb Symp Quant Biol. 50: 433-7
Jennewein S, Croteau R (2001) Taxol: biosynthesis, molecular genetics, and biotechnological applications. Appl. Microbiol. Biotechnol. 57:13-9.
Jennewein S, Wildung MR, Chau M, Walker K, Croteau R (2004) Random sequencing of an induced Taxus cell cDNA library for identification of clones involved in Taxol biosynthesis. Proc. Natl. Acad. Sci. USA 101:9149-9154.
Martin DM, Faldt J, Bohlmann J (2004) Functional characterization of nine Norway Spruce TPS genes and evolution of gymnosperm terpene synthases of the TPS-d subfamily. Plant Physiol. 135:1908-1927.
Mau CJ, West C (1994) Cloning of casbene synthase cDNA: evidence for conserved structural features among terpenoid cyclases in plants. Proc. Natl. Acad. Sci. USA 91:8497-8501.
McGarvey P, Kaper JM. (1991) A simple and rapid method for screening transgenic plants using the PCR. Biotechniques. Oct;11(4):428-32.
Peters RJ, Flory JE, Jetter R, Ravn MM, Lee HJ, Coates RM, Croteau RB (2000) Abietadiene synthase from grand fir (Abies grandis): characterization and mechanism of action of the "pseudomature" recombinant enzyme. Biochemistry 39:15592-15602.
Prisic S, Xu M, Wilderman PR, Peters RJ (2004) Rice Contains Two Disparate ent-Copalyl Diphosphate Synthases with Distinct Metabolic Functions. Plant Physiol. 136:4228-4236.
Rios JL, Recio MC (2005). Medicinal plants and antimicrobial activity. J Ethnopharmacol. 100:80-84.
Saroglou V, Karioti A, Demetzos C, Dimas K, Skaltsa H (2005) Sesquiterpene lactones from Centaurea spinosa and their antibacterial and cytotoxic activities. J Nat Prod. 68:1404-1407.
Schepmann HG, Pang J, Matsuda SP (2001) Cloning and characterization of Ginkgo biloba levopimaradiene synthase which catalyzes the first committed step in ginkgolide biosynthesis. Arch. Biochem. Biophys. 392:263-269.
Seo S, Seto H, Koshino H, Yoshida S, Ohashi Y (2003) A diterpene as an endogenous signal for the activation of defense responses to infection with tobacco mosaic virus and wounding in tobacco. Plant Cell. 15:863-873.
Severson RF, Johnson AW, Jackson DM (1985) Cuticular constituents of tobacco: factors affecting their production and their role in insect and disease résistance and smoke quality. Recent Advances in Tobacco Science 11:105-173.
Siemens J, and Schieder A (1996) Plant Tiss. Cult. Biotechnol. 2:66-75
Smith NA, Singh SP, Wang MB, Stoutjesdijk PA, Green AG, Waterhouse PM (2000) Total silencing by intron-spliced hairpin RNAs. Nature 407:319-20.
Sun TP, Kamiya Y (1994) The Arabidopsis GA1 locus encodes the cyclase ent-kaurene synthetase A of gibberellin biosynthesis. Plant Cell. 6:1509-1518.
Tissier A, Montané M-H (1999) Méthodes pour la production d'une banque de mutants et ses applications. Brevet Français n° 9913515.
Tissier A, Sallaud C, Rontein D (2004) Promoteurs végétaux et utilisations. Demande de brevet en France n° FR 0410799.
Trapp SC, Croteau RB (2001). Genomic organization of plant terpene synthases and molecular evolutionary implications. Genetics. 158:811-832.
Trombetta D, Castelli F, Sarpietro MG, Venuti V, Cristani M, Daniele C, Saija A, Mazzanti G, Bisignano G (2005). Mechanisms of antibacterial action of three monoterpenes. Antimicrob Agents Chemother. 49:2474-2478
Turner GW, Gershenzon J, Croteau R (2000) Development of peltate glandular trichomes of peppermint. Plant Phys. 124:665-680.
Wagner GJ, Wang E, Shepherd RW (2004) New approaches for studying and exploiting an old protuberance, the plant trichome. Annals of Botany 93:3-11
Wang E, Wang R, DeParasis J, Loughrin JH, Gan S, Wagner GJ (2001) Suppression of a P450 hydroxylase gene in plant trichome glands enhances natural-product-based aphid resistance. Nat Biotechnol. 19: 371-374.
Wang E, Wagner GJ (2003) Elucidation of the functions of genes central to diterpene metabolism in tobacco trichomes using posttranscriptional gene silencing. Planta 216:686-691.
Wang M-B, Graham MW, Waterhouse PM (1999) Methods and means for obtaining modified phenotypes. Demande de brevet WO9953050.
Wesley SV, Helliwell CA, Smith NA, Wang MB, Rouse DT, Liu Q, Gooding PS, Singh SP, Abbott D, Stoutjesdijk PA, Robinson SP, Gleave AP, Green AG, Waterhouse PM (2001) Construct design for efficient, effective and high-throughput gene silencing in plants. Plant J. 27:581-90.
Wilderman PR, Xu M, Jin Y, Coates RM, Peters RJ (2004) Identification of syn-pimara-7,15-diene synthase reveals functional clustering of terpene synthases involved in rice phytoalexin/allelochemical biosynthesis. Plant Physiol. 135:2098-2105.
Wildung MR, Croteau R (1994) A cDNA clone for taxadiene synthase, the diterpene cyclase that catalyses the committed step of taxol biosynthesis. J. Biol. Chem. 271:9201-9204.

**Tableau 1**

| Gène Abréviation | Nom du gène | Plantes | Promoteur (bp) | Plante transformée | Expression | **Références** |
|---|---|---|---|---|---|---|
| LTP3 | Lipid transfer | Cotton | 1548 | Tabac | Trichomes, épiderme | Liu et al., 2000, BBA, |
| | protein | | 1143 | | périphérique des | 1487:106111 |
| | | | 614 | | feuilles et tissus | |
| LTP6 | Lipid transfer | Cotton | 447 | Tabac | trichomes et cellules de | Hsu et al., 1999, Plant |
| | protein | | 272 | | guarde des stomates | science, 143:6370 |
| wax9D | Lipid transfer | Brassica | 972 | Tabac | Epidermes de feuilles, | Pyee and Kolattukudy, |
| | protein | oleracea | | | tiges et fleurs, pétales, | 1995, Plant J. 7:4559 |
| | | | | | sépales, ovules, et | |
| | | | | | trichomes | |
| LTP1 | Lipid transfer | Arabidopsis | 1149 | Arabidopsis | Cellules épidermiques | Thoma et al., 1994, |
| | protein | | | | de tissues divers | Plant Physiol. 105 |
| | | | | | | 3545 |
| CYC71D16 | CBTol | Tobacco | 1852 | Tabac | Trichomes | Wang et al., 2002, J.of |
| | hydroxylase | | | | | Exp. Bot. 18911897 |

**Tableau 2. Comparaison de l'efficacité de transformation entre N. Sylvestris et la lignée #804. Km : Kanamycin ; Nb : nombre ; #804: N Sylvestris contenant le transgène ihpTPS. Légendes des transgènes. FPS : gène (accession genbank AF048747) codant la farnesyl pyrophosphate synthase de tomate ; TPFPS : FPS avec un peptide transit d'adressage vers les chloroplastes. SSTLH : gene (accession Genbank AF279455) codant la germacrène synthase de tomate. TPSSTLH : SSTLH avec un peptide transit d'adressage vers les chloroplastes. Casbène : gène (accession Genbank L32134) codant la casbène synthase de ricin. GUSi : Gène uidA de Escherichia coli avec un intron pour empêcher l'expression dans les bactéries.**

| **EXP N°** | **Souche *d'agrobactérium*** | **Resistance T-DNA** | **Transgène** | **Nb disque foliaire** | **Nb plantes obtenues [N. sylvestris]** | **Nb plantes obtenues [#804]** |
|---|---|---|---|---|---|---|
| 1 | LBA4404 | Km | FPS+SSTLH | 100 | 4 | 41 |
| 2 | LBA4404 | Km | Casbène | 65 | 3 | 27 |
| 3 | LBA4404 | Km | GUSi/TPFPS/ TPSSTLH | 140 | 9 | 40 |

## Revendications

1. Méthode de production de terpène d'intérêt dans une plante présentant des trichomes glanduleux comprenant :
a) l'introduction dans une cellule de ladite plante d'une construction portant une cassette d'expression comprenant une séquence polynucléotidique codant pour une terpène synthase hétérologue permettant de synthétiser ledit terpène d'intérêt sous le contrôle d'un promoteur permettant une expression dans les trichomes;
b) la reconstitution d'une plante à partir de ladite cellule et la sélection des plantes transgéniques exprimant ladite terpène synthase ; et
c) la récolte du terpène d'intérêt contenu dans les trichomes des dites plantes transgéniques.

2. Méthode selon la revendication 1, dans laquelle l'expression de la terpène synthase est sous le contrôle d'un promoteur spécifique des trichomes.

3. Méthode selon la revendication 1 ou 2, dans laquelle ladite cassette d'expression comprend au moins une séquence activatrice « enhancer » liée de manière opérationnelle au promoteur.

4. Méthode selon l'une des revendications 1 à 3, dans laquelle la récolte du terpène d'intérêt contenu dans les trichomes des dites plantes transgéniques est effectuée par la récupération du terpène d'intérêt contenu dans l'exsudat des trichomes.

5. Méthode selon l'une des revendications 1 à 4, comprenant en outre le blocage de la voie de production de terpènes endogènes dans les trichomes, de préférence en bloquant l'expression des terpènes synthases endogènes.

6. Méthode selon la revendication 5, dans laquelle la plante transgénique sélectionnée en b) est croisée avec une plante transgénique dont la voie de production de terpènes endogènes est bloquée dans les trichomes.

7. Méthode selon l'une des revendications 1 à 6, dans laquelle ladite terpène synthase hétérologue est une diterpène synthase, de préférence la taxadiène synthase ou la casbène synthase.

8. Méthode selon l'une des revendications 1 à 6, dans laquelle ladite terpène synthase hétérologue est :
- une monoterpène synthase et la construction comprend en outre une séquence polynucléotidique codant pour une géranylpyrophosphate synthase sous le contrôle d'un promoteur permettant son expression dans les trichomes ; ou
- une sesquiterpène synthase et la construction comprend en outre une séquence polynucléotidique codant pour une farnésylpyrophosphate synthase sous le contrôle d'un promoteur permettant son expression dans les trichomes ; ou
- une triterpène synthase et la construction comprend en outre des séquences polynucléotidiques codant pour une farnésylpyrophosphate synthase, une squalène synthase et une squalène époxydase sous le contrôle d'un promoteur permettant leur expression dans les trichomes.

9. Méthode selon l'une des revendications 1 à 8, dans laquelle la plante est une plante de la famille des Asteracées, Cannabacées, Solanacées ou Lamiacées, de préférence le tabac, et en particulier *Nicotiana sylvestris.*

10. Méthode selon l'une des revendications précédentes, comprenant en outre l'introduction dans la cellule de ladite plante d'un transgène codant une enzyme de modification des terpènes.

11. Plante ou graine transgénique présentant des trichomes glanduleux **caractérisée en ce qu'**elle comprend une cassette d'expression contenant une séquence polynucléotidique codant pour une terpène synthase hétérologue permettant de synthétiser un terpène d'intérêt sous le contrôle d'un promoteur permettant une expression dans les trichomes, de préférence d'un promoteur spécifique des trichomes, et en outre facultativement au moins une séquence activatrice « enhancer » liée de manière opérationnelle au promoteur, et **en ce que** la voie de production de terpènes endogènes est bloquée dans les trichomes, de préférence en bloquant l'expression des terpènes synthases endogènes.

12. Plante ou graine transgénique selon la revendication 11, comprenant en outre un transgène codant pour une enzyme de modification des terpènes.

13. Plante ou graine transgènique selon l'une des revendications 11-12, **caractérisée en ce que** la plante est une plante de la famille des Asteracées, Cannabacées, Solanacées ou Lamiacées, de préférence du genre Nicotiana.

14. Utilisation d'une plante ou graine transgénique selon l'une des revendications 11 à 13 pour la production de terpène d'intérêt.

15. Méthode de récolte de terpènes hétérologues dans l'exsudat des trichomes d'une plante, comprenant a) la récolte de parties aériennes de la plante ; b) l'incubation de ces parties aériennes dans un solvant de type peu polaire ou apolaire ; et c) l'élimination du solvant.
